(19) Europäisches Patentamt
European Patent Office
Office européen des brevets

(11) Veröffentlichungsnummer: **0 243 904**
**B1**

(12) **EUROPÄISCHE PATENTSCHRIFT**

(45) Veröffentlichungstag der Patentschrift: **31.10.90**

(51) Int. Cl.$^5$: **C 07 D 295/08,** A 61 K 31/495

(21) Anmeldenummer: **87106033.1**

(22) Anmeldetag: **28.04.87**

(54) **Gegebenenfalls substituierte, 1-(2'-bis-(Phenyl)-methoxyr-äthyl)-4-(3'-bis-(phenyl)-methoxyr-propyl)-piperazine, Verfahren zu ihrer Herstellung und diese enthaltende Arzneimittel.**

(30) Priorität: **28.04.86 HU 175086**

(43) Veröffentlichungstag der Anmeldung:
**04.11.87 Patentblatt 87/45**

(45) Bekanntmachung des Hinweises auf die Patenterteilung:
**31.10.90 Patentblatt 90/44**

(84) Benannte Vertragsstaaten:
**AT BE CH DE FR GB IT LI LU NL SE**

(56) Entgegenhaltungen:
**US-A-2 988 551**

**JOURNAL OF THE AMERICAN PHARMACEUTICAL ASSOCIATION, Band 45, Nr. 5, Mai 1956, Seiten 317-320; C. RIFFKIN et al.: "Syntheses of amino ethers with potential pharmacological activity"**

(73) Patentinhaber: **Richter Gedeon Vegyészeti Gyár R.T.**
**Gyömröi ut 19-21**
**H-1475 Budapest X (HU)**

(72) Erfinder: **Tóth, Edit**
**Szabolcska M. u. 7**
**H-1114 Budapest (HU)**
Erfinder: **Kiss, Béla**
**Vöröshadsereg utja 197/c**
**H-2220 Vecsés (HU)**
Erfinder: **Törley, József**
**Katona J. u. 41**
**H-1137 Budapest (HU)**
Erfinder: **Pálosi, Eva, Dr**
**Vend u. 21**
**H-1025 Budapest (HU)**
Erfinder: **Hajdu, István**
**Tátra tér B/4**
**H-1205 Budapest (HU)**
Erfinder: **Szporny, László, Dr**
**Szabolcska M.u. 7**
**H-1114 Budapest (HU)**
Erfinder: **Groó, Dóra, Dr**
**Napraforgó u. 17**
**H-1021 Budapest (HU)**

**EP 0 243 904 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(72) Erfinder: **Lapis, Erzsébet**
**Abaliget u. 86**
**H-1172 Budapest (HU)**
Erfinder: **Laszlovszky, István, Dr**
**Bartók B. u. 16**
**H-1111 Budapest (HU)**

(74) Vertreter: **Beszédes, Stephan G., Dr.**
**Patentanwalt**
**Münchener Strasse 80a Postfach 1168**
**D-8060 Dachau (DE)**

**Beschreibing**

Die Erfindung betrifft neue, gegebenenfalls substituierte, 1-{2'-[bis-(Phenyl)-methoxy]-äthyl}-4-{3'-[bis-(phenyl)-methoxy]-propyl}-piperazine, ein Verfahren zu ihrer Herstellung und diese Verbindungen enthaltende Arzneimittel, insbesondere mit dopaminerger Wirkung auf das Zentralnervensystem.

Im Schrifttum ist die Herstellung von durch Benzhydryloxyalkylreste 1,4-disubstituierten Piperazinderivaten beispielsweise in den folgenden Veröffentlichungen beschrieben: CA *45*, 577 f; *50*, 4 405 b; *50*, 16 792 c; belgische Patentschriften 530 795 und 530 797 sowie US-Patentschrift 2 988 551. Die bekannten Verbindungen sind symmetrisch substituierte 1,4-bis-[2-(Benzhydryloxy)-äthyl]-piperazin- beziehungsweise 1,4-bis-[3-(Benzhydryloxy)-propyl]-piperazinderivate, die eine Antihistamin-Wirkung aufweisen.

Der Erfindung liegt die Aufgabe zugrunde, überlegene pharmakologische Wirkungen aufweisende neue durch Benxhydryloxyalkylreste 1,4-disubstituierte Piperazinderivate, ein Verfahren zu ihrer Herstellung und diese Verbindungen enthaltende Arzneimittel zu schaffen.

Das Obige wurde überaschenderweise durch die Erfindung erreicht.

Es wurde nämlich überraschenderweise festgestellt, daß die pharmakologische Wirkung der im folgenden festgelegten durch Benzhydryloxyalkylreste asymmetrisch 1,4-disubstituenten Piperazinderivate von denen der bekannten Piperazinderivate wesentlich abweicht.

Gegenstand der Erfindung sind daher, gegebenenfalls substituierte, 1-{2'-[bis-(Phenyl)-methoxy]-äthyl}-4-{3'-[bis-(phenyl)-methoxy]-propyl}-piperazine der allgemeinen Formel

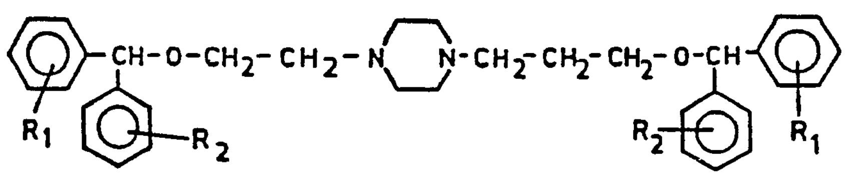

I,

worin

$R_1$ und $R_2$, die gleich oder verschieden sein können, für Wasserstoffatome, Halogenatome, Alkylreste mit 1 bis 4 Kohlenstoffatomen, Trihalogenmethylreste oder Alkoxyreste mit 1 bis 4 Kohlenstoffatomen stehen, sowie ihre Säureadditionssalze und optisch aktiven Isomere und Solvate.

Die erfindungsgemäßen 1-{2'-[bis-(Phenyl)-methoxy]-äthyl}-4-{3'-[bis-(phenyl)-methoxy]-propyl}-piperazine können 1 oder mehrere asymmetrische Kohlenstoffatome haben und daher in verschiedenen Stereoisomerformen vorkommen. Sie können also Basen, Säureadditionssalze, Racemate, getrennte optische Isomere und deren Gemische sowie Solvate, zum Beispiel Hydrate, sein.

Die erfindungsgemäßen 1-{2'-[bis-(Phenyl)-methoxy]-äthyl}-4-{3'-[bis-(phenyl)-methoxy]-propyl}-piperazine unterscheiden sich von den bekannten Verbindungen darin, daß sie in der angegebenen Weise am Piperazinring asymmetrisch 1,4-disubstituiert sind.

Vorzugsweise ist beziehungsweise sind das beziehungsweise die Halogenatom(e), für welche[s] $R_1$ und/oder $R_2$ stehen kann beziehungsweise können, und/oder die Halogenatome des beziehungsweise der Trihalogenmethylreste[s], für welche[n] $R_1$ und/oder $R_2$ stehen kann beziehungsweise können, Fluor und/ oder Chlor.

Es ist auch bevorzugt, daß der beziehungsweise die Alkylrest(e) mit 1 bis 4 Kohlenstoffatom(en), für welche[n] $R_1$ und/oder $R_2$ stehen kann beziehungsweise können, [ein] solche[r] mit 1 oder 2, ganz besonders 1, Kohlenstoffatom(en) ist beziehungsweise sind.

Ferner ist es bevorzugt, daß der beziehungsweise die Alkoxyrest(e) mit 1 bis 4 Kohlenstoffatom(en), für welche[n] $R_1$ und/oder $R_2$ stehen kann beziehungsweise können [ein] solche[r] mit 1 oder 2, ganz besonders 1, Kohlenstoffatom(en) ist beziehungsweise sind.

Weiterhin ist es bevorzugt, daß der beziehungsweise die an einem oder mehreren Phenylring(en) gegebenenfalls vorliegende(n) Substituent(en) in der beziehungsweise den 3- und/oder 4-Stellung(en) ist beziehungsweise sind.

Besonders bevorzugte erfindungsgemäße

1-{2'-[bis-(Phenyl)-methoxy]-äthyl}-4-{3'-[bis-(phenyl)-methoxy]-propyl}-piperazine sind

1-[2'-⟨bis-{4''-(Fluor)-phenyl}-methoxy⟩-äthyl]-4-[3'''-⟨bis-{4''''-(fluor)-phenyl}-methoxy⟩-propyl]-piperazin,

1-[2'-⟨{4''-(Methoxy)-phenyl}-{3'''-(trifluormethyl)-phenyl}-methoxy⟩-äthyl]-4-[3''''-⟨{4'''''-(methoxy)-phenyl}-{3''''''-(trifluormethyl)-phenyl}-methoxy⟩-propyl]-piperazin,

1-[2'-⟨bis-{4''-(Chlor)-phenyl}-methoxy⟩-äthyl]-4-[3'''-⟨bis-{4''''-(chlor)-phenyl}-methoxy⟩-propyl]-piperazin,

1-[2'-⟨{2''-(Methyl)-phenyl}-{4'''-(fluor)-phenyl}-methoxy⟩-äthyl]-4-[3''''-⟨{2'''''-(methyl)-phenyl}-{4''''''-(fluor)-phenyl}-methoxy⟩-propyl]-piperazin und 1-[2'-⟨Di-(phenyl)-methoxy⟩-äthyl]-4-[3''-⟨di-(phenyl)-methoxy⟩-propyl]-piperazin sowie ihre Säureadditionssalze.

Die Säureadditionssalze können solche mit anorganischen oder organischen Säuren sein. Zweckmäßig sind sie solche mit physiologisch brauchbaren Säuren. Beispiele für solche sind Halogenwasserstoffsäuren, wie Salzsäure und Bromwasserstoffsäure, Schwefelsäure, Phosphorsäuren, Ameisensäure, Essigsäure, Propionsäure, Oxalsäure, Glykolsäure, Maleinsäure, Fumarsäure, Bernsteinsäure, Weinäure, Ascorbinsäure, Citronensäure, Apfelsäure, Salicylsäure, Milchsäure, Benzoesäure, Zimtsäure, Asparaginsäure, Glutaminsäure, N-(Acetyl)-asparaginsäure, N-(Acetyl)-glutaminsäure, Alkylsulfonsäuren, wie Methansulfonsäure, Arylsulfonsäuren, wie Toluolsulfonsäure.

Gegenstand der Erfindung ist auch ein Verfahren zur Herstellung der erfindungsgemäßen Verbindungen, welches dadurch gekennzeichnet ist, daß,

a) gegebenenfalls substituierte, Benzhydrylhalogenide, Benzhydrole beziehungsweise ihre Alkoholate der allgemeinen Formel

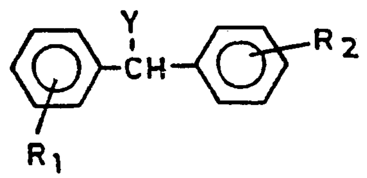

II,

worin

$R_1$ und $R_2$ die oben angegebenen Bedeutungen haben und

Y für ein Halogenatom, eine Hydroxygruppe oder einen Rest der Formel

—OM,

in welchletzterer M ein Alkalimetallatom bedeutet, steht,

mit, gegebenenfalls substituierten, 1-[2'-(Halogen)-äthyl]-, 1-[2'-(Alkylsulfonyloxy)-äthyl]-, 1-[2'-(Arylsulfonyloxy)-äthyl]-, 1-[2'-(Hydroxy)-äthyl]- beziehungsweise 1-[2'-(Metalloxy)-äthyl]-4-[3''-(halogen)-propyl]-piperazinen, -4-[3''-(alkylsulfonyloxy)-propyl]-piperazinen, -4-[3''-(arylsulfonyloxy)-propyl]-piperazinen, -4-[3''-(hydroxy)-propyl]-piperazinen, beziehungsweise -4-[3''-(metalloxy)-propyl]-piperazinen der allgemeinen Formel

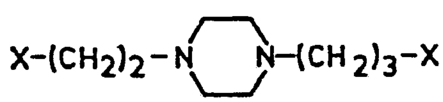

III,

worin

X für ein Halogenatom, einen Alkylsulfonyloxyrest, einen Arylsulfonyloxyrest, eine Hydroxygruppe oder einen Rst der allgemeinen Formel

—OM,

in welchletzterer M die obige Bedeutung hat, steht,

mit der Einschränkung, daß

α) X für eine Hydroxygruppe oder einen Rest der allgemeinen Formel —OM steht, wenn Y ein Halogenatom bedeutet, oder

β) X für ein Halogenatom, einen Alkylsulfonyloxyrest oder einen Arylsulfonyloxyrest steht, wenn Y eine Hydroxygruppe oder einen Rest der allgemeinen Formel —OM bedeutet,

umgesetzt werden, wobei die Reaktion, wenn von den Substituenten Y und X der eine für eine Gruppe der Formel —OM und der andere für ein Halogenatom steht, in wasserfreiem Medium in einem neutralen organischen Lösungsmittel, nämlich einem aliphatischen oder aromatischen Kohlenwasserstoff, einem aliphatischen oder cycloaliphatischen Äther oder Hexamethyl-phosphoramid oder einem Gemisch derselben, durchgeführt wird oder, wenn das Alkoholat der Verbindung der allgemeinen Formel (II) oder (III) mit einem Alkalimetalltertiäralkoholat gebildet wird, der entsprechende tertiäre Alkohol oder auch sein mit den obigen Lösungsmitteln gebildetes Gemisch als Lösungsmittel dient, oder,

b) gegebenenfalls substituierte, 1-{ω-[bis-[Phenyl]-methoxy]-alkyl}-piperazine der allgemeinen Formel

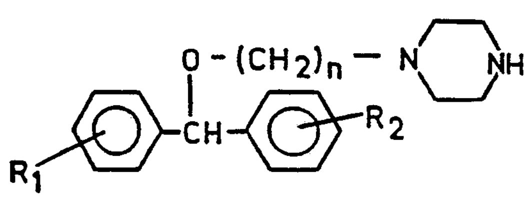

IV,

worin

$R_1$ und $R_2$ die oben angegebenen Bedeutungen haben und

n für 2 oder 3 steht,

mit, gegebenenfalls substituierten, [Di-(phenyl)]-[ω-(halogen)-alkoxy]-methanen, [Di-(phenyl)]-[ω-(alkyl-

sulfonyloxy)-alkoxy]-methanen beziehungsweise [Di-(phenyl)]-[ω-(arylsulfonyloxy)-alkoxy]-methanen der allgemeinen Formel

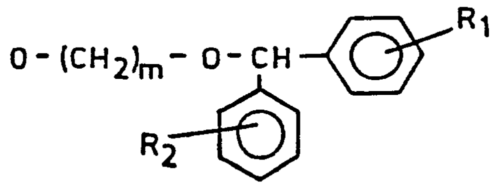

V,

worin

$R_1$ und $R_2$ die oben angegebenen Bedeutungen haben,
Q für ein Halogenatom, einen Alkylsulfonyloxyrest oder einen Arylsulfonyloxyrest steht und
m 2 oder 3 ist,

mit der Maßgabe, daß

m von n verschieden ist,

umgesetzt werden, wobei die Reaktion des monosubstituierten Piperazines der allgemeinen Formel (IV) und der Verbindung der allgemeinen Formel (V) in einem inerten organischen Lösungsmittel, nämlich in einem aromatischen Kohlenwasserstoff, einem Äther, einem Keton, einem Säureamid, einem Ester, einem niederen Alkanol oder einem halogenierten Kohlenwasserstoff durchgeführt wird und eine Reaktionstemperatur zwischen 40°C und dem Siedepunkt des Reaktionsgemsiches eingehalten wird, oder,

c) gegebenenfalls substituierte, Benzhydrylhalogenide, Benzhydrole beziehungsweise ihre Alkoholate der allgemeinen Formel II mit gegebenenfalls substituierten, 1-{ω-[bis-(Phenyl)-methoxy]-alkyl}-4-{ω-(halogen)-alkyl}-piperazinen, -4-{ω-(alkylsulfonyloxy)-alkyl}-piperazinen, -4-{ω-(arylsulfonyloxy)-alkyl}-piperazinen, -4-{ω-(hydroxy)-alkyl}-piperazinen beziehungsweise -4-{ω-(metalloxy)-alkyl}-piperazinen der allgemeinen Formel

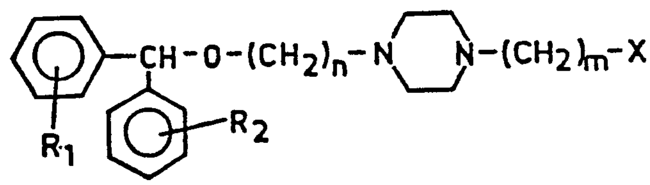

VI,

worin

$R_1$, $R_2$, X, m und n die obigen Bedeutungen haben,

mit den Maßgaben, daß

α) X für eine Hydroxygruppe oder einen Rest der allgemeinen Formel —OM steht, wenn Y ein halogenatom bedeutet, oder

β) X für ein Halogenatom, einen Alkylsulfonyloxyrest oder einen Arylsulfonyloxyrest steht, wenn Y eine Hydroxygruppe oder einen Rest der allgemeinen Formel —OM bedeutet, sowie ferner

γ) m von n verschieden ist,

umsetzt werden, wobei die unter a) eingehaltenen Reaktionsbedingungen eingehalten werden, sowie in an sich bekannte Weise gegebenenfalls die nach einer der Varianten a) bis c) erhaltenen 1-{2'-[bis-(Phenyl)-methoxy)-äthyl}-4-{3'-[bis-(phenyl)-methoxy]-propyl}-piperazine der allgemeinen Formel I mit anorganischen oder organischen Säuren in Säureadditionssalze überführt und/oder die erhaltenen Säureadditionssalze der 1-{2'-[bis-(Phenyl)-methoxy]-äthyl}-4-{3'-[bis-(phenyl)-methoxy]-propyl}-piperazine der allgemeinen Formel I in die entsprechenden freien 1-{2'-bis-(Phenyl)-methoxy]-äthyl}-4-{3'-[bis-(phenyl)-methoxy]-propyl}-piperazinbasen der allgemeinen Formel I oder in andere Säureadditionssalze überführt werden und/oder gegebenenfalls eine Spaltung der erhaltenen racemischen 1-{2'-[bis-(Phenyl)-methoxy]-äthyl}-4-{3'-[bis-(phenyl)-methoxy]-propyl}-piperazine der allgemeinen Formel I beziehungsweise von deren Säureadditionssalzen beziehungsweise eine Racemisierung der entsprechenden optisch aktiven Verbindungen in Stereoisomere vorgenomen wird.

Als Alkalimetall, für welches M stehen kann, wird bevorzugt Lithium, Kalium oder Natrium verwendet.

Bei der Durchführung der Ausführungsform a) des erfindingsgemäßen Verfahrens wird vorzugsweise in inerter Gasatmosphäre, z.B. in Stickstoff- oder Argonatmosphäre, gearbeitet. Beispiele für als Lösungsmittel verwendbare aliphatische oder aromatische Kohlenwasserstoffe sind Hexan, Ligroin, Benzol, Toluol und Xylol und für als Lösungsmittel verwendbare aliphatische oder cycloaliphatische Äther Dipropyläther, Dibutyläther, Tetrahydrofuran, Dioxan und Dimethylsulfoxyd und deren Gemische.

Für den Fall, dass einer von den Substituenten Y und X für Hydroxylgruppe, der andere für Halogenatom (vorzugsweise Chlor- oder Bromatom) oder Alkyl- oder Arylsulfonylgruppe (vorzugsweise Methansulfonyloxy- oder p-Tosyloxygruppe) steht, wird die Reaktion vorzugsweise in Gegenwart einer zur Bindung der freiwerdenden Säure geeigneten anorganischen oder tertiären organischen Base durchgeführt. Als anorganische Base können zum Beispiel Alkalimetall- oder Erdalkalimetallcarbonate (wie Kaliumcarbonat, Natriumcarbonat), als tertiäre organische Base zum Beispiel Pyridin, Triäthylamin, Tripropylamin verwendet werden.

Auf das Verfahren c) können die oben beschriebenen Bedingungen angewendet werden.

Bei der Variante b) sind Beispiele für als Lösungsmittel verwendbare aromatische Kohlenwasserstoffe Benzol, Toluol und Xylol, für als Lösungsmittel verwendbare Äther Dibutyläther und Dioxan, für als Lösungsmittel verwendbare Ketone 4-Methyl-2-pentanon, für als Lösungsmittel verwendbare Säureamide N,N-Dimethylformamid, für als Lösungsmittel verwendbare Ester Äthylacetat, für als Lösungsmittel verwendbare niedere Alkanole Propanol und Butanol und für als Lösungsmittel verwendbare halogenierte Kohlenwasserstoffe Chlorbenzol. Zur Bindung der bei der Reaktion entstandenen Säure kann eine entsprechende organische oder anorganische Base (wie beispielsweise Träthylamin, Tripropylamin, 4-Dimethylamino-pyridin, Kaliumcarbonat, Natriumcarbonat verwendet werden. Als Katalysator kann zur Förderung der Reaktion noch eine kleine Menge Metalljodid (zum Beispiel Natrium- oder Kaliumjodid) zum Reaktionsgemisch gegeben werden. Zur Beschleunigung der Reaktion wird diese bei einer Temperatur zwischen 40°C und dem Siedepunkt des Reaktionsgemisches durchgeführt. Das Reaktionsprodukt wird bei jedem Verfahren aus dem Reaktionsgemisch isoliert, zum Beispiel durch Behandlung des Reaktionsgemisches mit Wasser, Extraktion des Produktes mit einem organischen Lösungsmittel und — wenn notwendig — Reinigung mit bekannten Verfahren (zum Beispiel Destillation, Chromatographie, Kristallisation).

Das Überführen der erfindungsgemäßen 1-{2'-[bis-(Phenyl)-methoxy]-äthyl}-4-{3'-[bis-(phenyl)-methoxy]-propyl}-piperazine der allgemeinen Formel I in Säureadditionssalze kann nach an sich bekannten Verfahrensweisen durchgeführt werden. Die Salzbildung kann zum Beispiel so vor sich gehen, dass zu der mit inertem Lösungsmittel (zum Beispiel Äthanol) bereiteten Lösung der Verbindung der allgemeinen Formel (I) die entsprechende Säure gegeben und das Salz vorzugsweise mit einem sich nicht mit Wasser vermischenden organischen Lösungsmittel (wie zum Beispiel Diäthyläther) ausgefällt wird.

Die Ausgangsstoffe sind bekannt oder können analog zu in der Literatur beschriebenen Verfahren hergestellt werden. Die Benzhydrole der allgemeinen Formel (II) können zum Beispiel aus den entsprechenden Carbonylverbindungen durch die Reaktion mit Grignard- Reagens, beispielsweise nach der Methode, die von M. S. Kharasch und Mitarbeitern beschrieben wurde (Grignard reaktions of nonmetallic substances, Ed. Prentice-Hall Inc., Seiten 138—143 [1954]) oder durch die Reduktion der entsprechenden Benzophenone mit Alkalimetallborhydrid (E. Schenker: Angewandte Chemie *73*, 81—90 [1961]) synthetisiert werden.

Die Herstellung der Alkohole der allgemeinen Formel (III) wird in der britischen Patentschrift Nr. 807 750 beziehungsweise CA *53*, 22027 d beschrieben. Die Alkohole der allgemeinen Formel (VI) können beispielsweise hergestellt werden, indem die Alkoholate der allgemeinen Formel (II) unter den beim Verfahren a) angegebenen Bedingungen mit 1-(2-Hydroxyäthyl)-4-(3-chlorpropyl)piperazin oder mit 1-(3-Hydroxypropyl)-4-(2-chloräthyl)piperazin, welche zum Beispiel nach der Methode von L. Toldy (Acta Chim. Acad. Sci. Hung. *42*, 351—7 [1964]) herstellbar sind, umgesetzt werden.

Aus den Alkoholen der allgemeinen Formeln (II), (III) und (VI) können die Alkoholate zum Beispiel nach den von Houben-Weyl (Methoden der Organischen Chemie, Band VI/2, Seiten 6—34 [1963]) beschriebenen Methoden gewommen werden.

Die Verbindungen der allgemeinen Formeln (II), (III), (V) und (VI) können zum Beispiel aus den entsprechenden Alkoholen mit gemäss dem Stand der Technik bekannten Verfahren hergestellt werden. Die Halogenide können aus den Alkoholen durch die Reaktion mit geeigneten Halogeniermitteln, beispielsweise mit Thionylchlorid, Thionylbromid, Phosphorpentachlorid oder -bromid bzw. Phosphoroxychlorid, gewonnen werden. Die eine Alkylsulfonyloxy- oder Arylsulfonyloxygruppe enthaltenden Ester, zum Beispiel die Methansulfonate oder p-toluolsulfonate, kann man durch die Reaktion von alkoholen und Methansulfonylchlorid oder p-Toluolsulfonylchlorid erhalten. Die Verbindungen der allgemeinen formel (V) können zum Beispiel nach dem von Sugasawa und Mitarbeitern beschriebenen Verfahren (Org. Synthesis *33*, 11 [1953]) hergestellt werden.

Die monosubstituierten Piperazin-Derivate der allgemeinen Formel (IV) können unter anderem hergestellt werden, indem die Verbindungen der allgemeinen Formel (V) mit im Überschuss verwedetem Piperazin ummgesetzt werden (Ind. Chim. Belge *22*, 416 [1957]), sie können aber auch durch das von Kiichi Fujii und Mitarbeitern beschriebene Verfahren (J. Pharm. Soc. Japan *74*, 1049—51 [1954]) synthetisiert werden.

Gegenstand der Erfindung sind auch Arzneimittel, welche 1 oder mehr der erfindungsgemäße Verbindungen als Wirkstoff(en), gegebenenfalls zusammen mit 1 oder mehr üblichen pharmazeutischen Träger- und/oder Hilfsstopff(en), enthalten.

Die erfindungsgemäßen Verbindungen haben nämlich überlegene pharmakologische Wirkungen, insbesondere eine starke, selektive dopaminerge Wirkung auf das Zentralnervensystem und sind als solche zur Behandlung von krankhaften Zuständen, die sich infolge der Degeneration und/oder Hypofunktion des dopaminergen Systems einstellen (wie zum Beispiel bestimmte neuroendokrine Erkrankungen, Depression, "Altern", Parkinsonismus und Impotenz), geeignet. Kurz ausgedrückt handelt es sich um die Verwendung zur Behandlung von Krankheiten, die mit einer Senkung des Dopaminspiegels einhergehen.

Die dopaminerge Wirkung der erfindungsgemässen Verbindungen der allgemeinen Formel (I) beziehungsweise ihrer Salze wurde an Mäusen und Ratten in "in vivo"-Versuchen untersucht.

a) Messen der Veränderung der lokomotirschen Aktivität

Dopaminerge Stoffe beeinflussen die mit L—DOPA induzierte Hypermotilität (Plotnikoff N. P. und Mitarbeiter: The Thyroid Axis Drug and Behaviour, Raven Press, N.Y. 103—113 [1974]).

Für die Untersuchungen wurden 160—180 g schwere männliche Hann-Wistar-Ratten verwendet. Aus jeweils 5 Tieren bestehenden Gruppen wurden intraperitoneal mit 40 mg/kg Pargylin [N-Methyl-N-(2-propinyl)benzylamin]behandelt, dann wurden die zu untersuchenden Stoffe nach 60 Minuten in 0,5%-iger Tween®-Lösung in einer Dosis von 30 mg/kg oral verabreicht. Die Kontrollgruppen wurden mit Placebo (0,5% Tween enthaltende destillierte Wasserlösung, 10 ml/kg) behandelt. 30 Minuten nach Gabe der Stoffe erhielten die Tiere Intraperitoneal 100 mg/kg L—DOPA. Die lokomotorische Aktivität der Tiere wurde nach der Behandlung 3 Stunden lang mit einem VKI-Fünfkanal-Aktivitätsmesser (Typ: UPAMS®—01) registriert. (Während der messzeit wurde die Zeit der aktiven Bewegung der Tiere registriert.) Die Ergebnisse sind in % der Kontrolle in der Tabelle 1 angegeben.

Abkürzungen:

n = Anzahl der Tiere
i. p. = intraperitoneal
p. o. = oral
L—DOPA = L-β-3,4-Dihydroxy-phenylalanin
$\bar{x} \pm SE$ = Durchschnittswert ± Standardfehler

Die in der Tabelle vorkommenden Verbindungen sind:

A = 1-[2'-⟨bis-{4''-(Fluor)-phenyl}-methoxy⟩-äthyl]-4-[3'''-⟨bis-{4''''-(fluor)-phenyl}-methoxy⟩-propyl]-piperazindimaleat,

B = 1-[2'-⟨{4''-(Methoxy)-phenyl}-{3'''-(trifluormethyl)-phenyl}-methoxy⟩-äthyl]-4-[3''''-⟨{4'''''-(methoxy)-phenyl}-{3''''''-(trifluormethyl)-phenyl}-methoxy⟩-propyl]-piperazindimaleat,

C = 1-[2'-⟨bis-{4''-(Chlor)-phenyl}-methoxy⟩-äthyl]-4-[3'''-⟨bis-{4''''-(chlor)phenyl}-methoxy⟩-propyl]-piperazindimaleat,

D = 1-[2'-⟨{2''-(Methyl)-phenyl}-{4'''-(fluor)-phenyl}-methoxy⟩-äthyl]-4-[3''''-⟨{2'''''-(methyl)-phenyl}-{4''''''-(fluor)-phenyl}-methoxy⟩-propyl]-piperazindimaleat,

E = 1-[2'-⟨Di-(phenyl)-methoxy⟩-äthyl]-4-[3''-⟨di-(phenyl)-methoxy⟩-propyl]-piperazindimaleat.

TABELLE 1

| Verbindung | Dosis mg/kg p.o. | Lokomotorische Aktivität Gesamtbewegung/3 h in % der Kontrolle | n |
|---|---|---|---|
| A | 30 | 175 ± 9,3 | 15 |
| B | 30 | 151 ± 7,1 | 15 |
| C | 30 | 202 ± 9,8 | 15 |
| D | 30 | 157 ± 8,4 | 15 |
| Placebo-Kontrolle | — | 100 ± 10,4 | 15 |

Placebo-Kontrolle $\bar{x} \pm$ S.E. = 1986 ± 208,5 sec.

Aus den Ergebnissen ist ersichtlich, dass die erfindungsgemässen Verbindungen die durch L-DOPA ausgelöste Hyperaktivität signifikant erhöhen und als solche in Zuständen mit Dopaminmangel eine bedeutende verbessernde Wirkung aufweisen.

b) Hemmen der neurotoxischen Wirkung von 1-Methyl-4-phenyl-1,2,3,6-tetrahydropyridin (MPTP)

Über das MPTP wurde bereits 1979 geschrieben, dass es bei Menschen und bei Affen eine Degeneration der Neuronen des dopaminergen Systems verursacht [siehe Davis G. C. und Mitarbeiter: Psychiat. Res. *1*, 249—254 (1979); Burns R. S. und Mitarbeiter: Proc. Natl. Acad. Sci. (USA) *80*, 4546—4550 (1983)], danach wurde die selektive dopaminerge, schädliche Wirkung der Verbindung auch an Mäusen nachgewiesen [zum Beispiel Hallman H. und Mitarbeiter: Eur. J. Pharmacol. *97*, 133—136 (1984); Pileblad E. und Mitarbeiter: Neuropharmacol. *24*, 689—698 (1985)]. Die mit MPTP an Versuchstieren hervorgerufene selektive dopaminerge, schädigende Wirkung kann als analoger Vorgang zu den Degenerations- und Hypofunktionserkrankungen des humanen dopaminergen Systems betrachtet werden und ist daher als Modell für die Untersuchung von Verbindungen, die in der Therapie zur Behandlung von mit der krankhaften Funktion des dopaminergen Systems zusammenhängenden Krankheiten dienen können,

geeignet [Bradbury A. J. und Mitarbeiter: The Lancet 1444—45 (1985); Przuntek H. und Mitarbeiter: Life Sci. *37*, 1195—1200 (1985)].

Für die Untersuchungen wurden 20—25 g schwere männliche CFY-Mäuse verwendet (LATI, Gödöllö). Die Verbindungen wurden in 1%-iger Tween® 80-Lösung homogenisiert und den Tieren 1 Stunde vor dem MPTP auf die in der Tabelle 2 angegebene Weise in einer Dosis von 0,1 mMol/kg verabreicht. Das MPTP erhielten die Tiere frisch in physiologischer Salzlösung aufgelöst, in einer Dosis von 70 mg/kg subcutan. Dann wurden die Tiere 72—96 Stunden nach der Gabe des MPTP enthauptet, das Gehirn wurde entfernt, in eiskalter physiologischer Salzlösung abgekühlt, das Striatum wurde herauspräpariert und über Trockeneis eingefroren.

Das Gewicht der Gewebe wurde (in gefrorenem Zustand) gemessen, dann wurden die Gewebe in 1 ml 0,4n eiskaltes, 0,5% $Na_2S_2O_5$, 0,25% $Na_2EDTA$ und 100 ng N-Methyldopamin (Internationalstandard zur Bestimmung der Catecholamine) enthaltender Perchlorsäure mit einem Ultra-Turrax®-Gerät homogenisiert. Das Homogenisat wurde 10 Minuten lang bei +4°C mit 20 000 g zentrifugiert, dann wurden dem Überstand 0,8 ml entnommen. Nach Zugabe von 20 mg aktiviertem $Al_2O_3$ wurde der pH mit 0,8 ml 0,5 m Tris-Lösung auf 8 eingestellt, und die Röhrchen wurden 20 Minuten lang geschüttelt. Man liess das Aluminiumoxyd sich absetzen, der Überstand wurde abgesaugt, dann 3-mal mit 5 ml destilliertem Wasser gewaschen. Die am Aluminiumoxyd adsorbierten Catecholamine wurden mit 1 ml 0,05 n Perchlorsäure eluiert. Aus einem Teil des Eluats wurde mittels Hochdruck-Flüssigkeitschromatographie mit Hilfe eines elektrochemischen Detektors Dopamin bestimmt (Laborpumpe MIM OE—230, 4 × 150 mm Nucleosil 5 C—18 Analysen- und 4 × 20 mm Nucleosil 5 C—18-Vorlaufsäule, mit Glassy-carbon-Arbeitselektrode und $Ag/AgCl_2$-Referenzelektrode ausgestatteter elektrochemischer Detektor, Eltron®-Potentiostat, Zweikanalrecorder LKB 2110, Oxydationspotential: 600 mV, mobile Phase: 0,1 m $NaH_2PO_4$, 1 mM $Na_2EDTA$, 1 mM Octansulfonsäure, die 8,4% Acetonitril enthält, Fliessgeschwindigkeit: 1 ml/min).

Mit der angewandten Methode kann 50—60%-ige Senkung des Dopaminspiegels des Striatums erreicht werden. Der Schutz der MPTP-induzierten Dopaminfreisetzung wurde wie folgt errechnet:

$$\text{Hemmung \%} = \frac{\text{(Verbindung + MPTP-behadelt)} - \text{(MPTP-behandelt)}}{\text{(Kontrolle)} - \text{(MPTP-behandelt)}} \times 100$$

Als Vergleichssubstanz wurde Trihexyphenidyl-hydrochlorid in einer i.p. Dosis von 10 mg/kg verwendet. Dies ist weniger als 0,1 mMol/kg; bei Dosen von mehr als 10 mg/kg verendeten die Tiere. Die Ergebnisse sind in der Tabelle 2 angegeben.

TABELLE 2

| Verbindung | Dosis mMol/kg | Art der Verabreichung | Hemmung der mit MPTP induzierten DA-Senking % | n |
|---|---|---|---|---|
| A | 0,1 | i.p. | 94 | 7 |
| | | p.o. | 87 | 7 |
| E | 0,1 | p.o. | 69 | 7 |
| Trihexyphenidyl-HCl | | i.p. | 7 | 7 |

Hieraus ist ersichtlich, dass die erfindungsgemässen Verbindungen der allgemeinen Formel (I) Tieren oral und/oder intraperitoneal von der Behandlung mit MPTP verabreicht die neurotoxische, den Dopaminspiegel senkende Wirkung des MPTP in grossem Ausmass oder völlig ausschalten können. Die Verbindungen haben eine günstig niedrige Toxizität. Auf Grund der oben genannten Tatsachen sind die erfindungsgemässen Verbindungen ein wertvolles therapeutisches Mittel zur Beeinflussung von Krankheitsbildern, bei denen eine Degeneration des dopaminergen Systems oder — aus anderen Gründen — dopaminerge Hypofunktion besteht.

Die erfindungsgemäßen Verbindungen können zu Arzneimittelpräparaten verarbeitet werden. Die arzneimittelpräparate können oral, rektal und/oder parenteral verabreicht werden. Zur oralen Verabreichung kann das Präparat in Form von Tabletten, Dragées oder Kapseln hergestellt werden. Bei der Herstellung von oralen Präparaten kann als Füllstoff zum Beispiel Milchzucker oder Stärke, als Binde- oder Granuliermittel zum Beispiel Gelatine, Carboxymethylzellulose-natrium, Methylzellulose, Polyvinylpyrrolidon oder Stärkekleister verwendet werden. Als zerfallsfördernde Mittel werden in erster Linie Kartoffelstärke oder mikrokristalline Zellulose zugegeben, es kann aber beispielsweise auch Ultraamylopectin oder Formaldehydcasein verwendet werden. Als antiadhäsive und Gleitstoffe kann man zum Beispiel Talk, kolloidale Kieselsäure, Stearin, Kalzium- und Magnesiumstearat verwenden.

Die Tabletten können zum Beispiel durch Granulieren und darauffolgendes Pressen hergestellt werden. Die miteinander vermischten Wirk- und Füllstoffe sowie gegebenenfalls ein Teil der zerfallsfördernden Zusätze werden mit der wässrigen, alkoholischen oder wässrigalkoholischen Lösung der Bindemittel in einer entsprechenden Einrichtung granuliert, dann wird das Granulat getrocknet. Danach gibt man zu dem getrockneten Granulat die übrigen zerfallsfördernden, Gleit- und antiadhäsiven Hilfsstoffe, und das Gemsich wird zu Tabletten gepresst. Gegebenenfalls werden die Tabletten zur Erleichterung der Dosierung mit einer Teilungsrille versehen. Die Tabletten können auch direkt durch Pressen aus dem Gemisch des Wirkstoffes und geeigneter Hilfsstoffe hergestellt werden. Die Tabletten können gewünschtenfalls unter Verwendung von bei der Arzneimittelherstellung allgemein gebräuchlichen Schutz-, Geschmacks- beziehungsweise Farbstoffen, wie beispielsweise Zucker, Zellulosederivaten (Methyl- oder Äthylzellulose, Carboxymethylzellulose-natrium), Polyvinylpyrrolidon, Kalziumphosphat, Kalziumcarbonat, Lebensmittelfarbstoffen, Lebensmittelfarblacken, Aromastoffen, Eisenoxydpigmenten dragiert werden.

Bei der Herstellung von Kapseln wird das Gemisch von Wirk- und Hilfsstoffen in Kapseln gefüllt.

Zur rektalen Anwendung wird das Präparat in Zäpfchenform hergestellt. Die Zäpchen enthalten ausser dem Wirkstoff Trägerstoffmasse, sogenannte Adeps pro suppositorio. Als Trägerstoff können Pflanzenfette, wie beispielsweise erhärtete pflanzliche Öle, Triglyceride von $C_{12-18}$-Fettsäuren, vorzugsweise unter dem geschützten Namen Witepsol® bekannte Trägerstoffe, verwendet werden. Der Wirkstoff wird in der geschmolzenen Trägerstoffmasse homogen verteilt, und es werden Zäpfchen gegossen.

Für die parenterale Verabreichung wird das Präparat in Form von Injektionen hergestellt. Bei der Herstellung von Injektionslösungen werden die Wirkstoffe gegebenenfalls in Gegenwart von Lösungsvermittlern [wie bveispielsweise Polyoxäthylensorbitanmonolaurat, -monooleat oder -monostearat (Tween® 20, Tween® 60, Tween® 80)] in destilliertem Wasser und/oder in verschiedenen organischen Lösungsmitteln (wie beispielsweise in Glykoläthern) gelöst. Die Injektionslösung kann ausserdem noch verschiedene Hilfsstoffe, und zwar Konserviermittel (wie zum Beispiel Benzylalkohol, p-Hydroxybenzoesäure-methyl- oder -propylester, Benzalkoniumchlorid oder Phenyl-mercuriborat), weiterhin Antioxydantien (wie beispielsweise Ascorbinsäure, Tocopherol, Natriumpyrosulfat) und gegebenenfalls komplexbildende Stoffe zur Bindung von Metallspuren (wie zum Beispiel Äthylendiamintetraacetat), weiterhin den pH einstellende und Pufferstoffe sowie gegebenenfalls Lokalanästhetica (wie beispielsweise Lidocain) enthalten. Die das erfindungsgemässe Arzneimittelpräparat enthaltende Injektionslösung wird vor dem Füllen in die Ampullen filtriert und nach dem Füllen sterilisiert.

Die tägliche Dosis ist vom Zustand des Kranken und von der zu behandelnden Anomalie abhängig. Bei oraler Verabreichung beträgt die gebräuchliche Dosis bei Erwachsenen 5—200 mg.

Die Herstellung der Piperazin-Derivate der allgemeinen Formel (I) wird in den folgenden Beispielen veranschaulicht.

Beispiel 1

1-[2'-⟨bis-{4''-(Fluor)-phenyl}-methoxy⟩-äthyl]-4-[3'''-⟨bis-{4''''-(fluor)-phenyl}-methoxy⟩-propyl]-piperazindimaleat

Die Suspension von 2,5 g 60%-iger öliger Natriumhydrid-Dispersion, 13,7 g 4,4'-Difluorbenzhydrol und 64 ml wasserfreiem Toluol wird in Stickstoffatmosphäre unter Rühren 30 Minuten lang am Rückfluss gekocht, danach wird die mit 14 ml wasserfreiem Toluol bereitete Lösung von 6,8 g 1-(2-Chloräthyl)-4-(3-chlorpropyl)-piperazin zugetropft. Es wird weitere 2 Stunden gekocht, dann wird das Reaktionsgemisch abgekühlt und Wasser zugegeben. Die organische Phase wird abgetrennt, mit Wasser gewaschen, über wasserfreiem Kaliumcarbonat getrocknet und bei verringertem Druck eingedampft. Die Benzollösung des Rückstandes wird durch eine Aluminiumoxydschicht filtriert, und nach Eindampfen wird in äthanolischer Lösung eine äthanolische Maleinsäurelösung zugegeben. Der Schmelzpunkt des kristallinen Dimaleatsalzes beträgt 189—190°C.

Aus dem Dimaleatsalz wird die Base mit verdünnter wässriger Ammoniumhydroxydlösung freigesetzt.

Elementaranlyse der Base, auf die Formel $C_{35}H_{36}F_4N_2O_2$ berechnet:

C = 70,93%; H = 6,12%; F = 12,82%; N = 4,73%;

gefunden: C = 71,01%; H = 6,03%; F = 12,70%; N = 4,86%.

Analog zu dem im obigen Beisiel beschriebenen Verfahren wird die folgende Verbindung hergestellt:

1-[2'-⟨{4''-(Methoxy)-phenyl}-{3'''-(trifluormethyl)-phenyl}-methoxy⟩-äthyl]-4-[3''''-⟨{4'''''-(methoxy)-phenyl}-{3''''''-(trifluormethyl)-phenyl}-methoxy⟩-propyl]-piperazindimaleat; Schmelzpunkt: 166—167°C.

Die Herstellung erfolgt durch die Reaktion von 1-(2-Hydroxyäthyl)]-4-(3-hydroxypropyl)-piperazin-dimethansulfonat und dem Natriumalkoholat von 3-(Trifluormethyl)-4-methoxybenzhydrol.

Elementaranlyse der Base, auf die Formel $C_{39}H_{42}F_6N_2O_4$ berechnet:

C = 65,35%; H = 5,91%; F = 15,90%; N = 8,93%;

gefunden: C = 65,50%; H = 6,00%; F = 15,98%; N = 8,72%.

Beispiel 2

1-[3'-⟨{bis-{4''-(Fluor)-phenyl}-{phenyl}-methoxy⟩-propyl]-4-[2'''-⟨{di-(phenyl)}-methoxy⟩-äthyl]-piperazin

10,1 g Benzhydrol und 6,2 g Kalium-tert-butylat werden in 100 ml wasserfreiem tert-Butylalkohol in Argonatmosphäre 30 Minuten lang unter Rückfluss gekocht, dann wird die mit 40 ml wasserfreiem tert-Butylalkohol bereitete Lösung von 19,6 g 1-[3-(4-Fluorphenyl)phenylmethoxy]-propyl]-4-[2-chloräthyl]-piperazin zugetropft. Es wird eine weitere Stunde gekocht, dann wird das Reaktionsgemisch bei verringertem Druck auf ein Volumen von etwa 40 ml eingedampft, es wird Wasser zugegeben und mit Benzol extrahiert. Die Benzolphase wird über wasserfreim Magnesiumsulfat getrocknet, das Benzol wird bei verringertem Druck abdestilliert, und der Rückstand wird im Vakuum fraktioniert. Der Siedepunkt der Titelverbindung beträgt 247—250°C/0,01 mm Hg.

Elementaranalyse, auf die Formel $C_{35}H_{39}FN_2O_2$ berechnet:

C = 78,03%; H = 7,30%; F = 3,53%; N = 5,20%;

gefunden: C = 78,21%; H = 7,11%; F = 3,69%; N = 5,37%.

Beispiel 3

1-[2'-⟨{2''-(Methyl)-phenyl}-{4'''-(fluor)-phenyl}-methoxy⟩-äthyl]-4-[3''''-⟨{2'''''-(methyl)-phenyl}-{4''''''-(fluor)-phenyl}-methoxy⟩-propyl]-piperazindimaleat

Die mit 120 ml Dimethylformamid bereitete Lösung von 9,4 g 1-(2-Hydroxyäthyl)-4-(3-hydroxypropyl)-piperazin, 29,3 g 2-Methyl-4'-fluorbenzhydryl-chlorid und 24 ml Tripropylamin wird unter Rühren 8 Stunden lang am Rückfluss gekocht, dann wird das Reaktionsgemisch bei verringertem Druck eingedampft. Zum Rückstand wird Wasser gegeben, und es wird mit Benzol extrahiert. Die organische Phase wird mit Wasser gewaschen, über wasserfreiem Nariumsulfat getrocknet, und das Lösungsmittel wird im Vakuum abdestilliert. Der rückstand wird in Methanol gelöst, mit Knochenkohle geklärt, filtriert und zum Filtrat wird ätherische Maleinsäurelösung gegeben. Der abgesonderte Niederschlag wird filtriert und aus Methanol umkristallisiert. Der Schmelzpunkt des Dimaleats beträgt 178—179.5°C.

Elementaranalyse der Base, auf die Formel $C_{37}H_{42}F_2N_2O_2$ berechnet:

C = 75,99%; H = 7,24%; F = 6,50%; N = 4,79%;

gefunden: C = 76,20%; H = 7,33%; F = 6,63%; N = 4,88%.

Beispiel 4

1-[3'-⟨{4''-(Fluor)-phenyl}-{phenyl}-methoxy⟩-propyl]-4-[2'''-⟨{4''''-(fluor)-phenyl}-{phenyl}-methoxy⟩-äthyl]-piperazindimaleat

6,6 g 1-[3-[(4-Fluorphenyl)phenylmethoxy]-propyl]-piperazin, 6,6 g 2-[(4-Fluorphenyl)phenylmethoxy]-äthylchlorid, 4,1 g wasserfreies pulverisiertes Kaliumcarbonat und 0,5 g Kaliumjodid werden in 70 ml Methylisobutylketon unter Rühren 17 Stunden lang am Rückfluss gekocht. Nach Abkühlen wird das Reaktionsgemisch bei verringertem Druck eingedampft. Zum Rückstand werden Wasser und Chloroform gegeben. Die organische Phase wird abgetrennt, mit Wasser gewaschen, dann über wasserfreiem Magnesiumsulfat getrocknet und im Vakuum eingedampft. Der Rückstand wird in wasserfreiem Äther gelöst, und das Salz wird mit der ätherischen Lösung von Maleinsäure abgetrennt. Nach Umkristallisieren aus Methanol beträgt der Schmelzpunkt des Dimaleatsalzes der Titelverbindung 176—177°C.

Elementaranalyse der Base, auf die Formel $C_{35}H_{38}F_2N_2O_2$ berechnet:

C = 75,51%; H = 6,88%; F = 6,83%; N = 5,03%;

gefunden: C = 75,55%; H = 7,11%; F = 6,65%; N = 5,28%.

Analog zu dem im obigen Beispiel beschriebenen Verfahren wird die folgende Verbindung hergestellt:

1-[2'-⟨Di-{phenyl}-methoxy⟩-äthyl]-4-[3''-⟨di-{phenyl}-methoxy⟩-propyl]-piperazindimaleat; Schmelzpunkt: 175—176°C.

Die Herstellung erfolgt durch die Reaktion von 3-(Diphenylmethoxy)propyl-p-toluolsulfonat und 1-[2-(Diphenylmethoxy)äthyl]piperazin.

Elementaranalyse der Base, auf die Formel $C_{35}H_{40}N_2O_2$ berechnet:

C = 80,73%; H = 7,74%; N = 5,38%;

gefunden: C = 80,88%; H = 7,66%; N = 5,50%.

Beispiel 5

1-[2'-⟨bis-{4''-(Chlor)-phenyl}-methoxy⟩-äthyl]-4-[3'''-⟨bis-{4''''-(chlor)-phenyl}-methoxy⟩-propyl]-piperazindimaleat

Die mit 100 ml wasserfreiem Xylol bereitete Lösung von 5,7 g 1-(2-Hydroxyäthyl)-4-(3-hydroxypropyl)-piperazin, 14,3 ml Tripropylamin und 20,4 g 4,4'-Dichlorbenzhydrylchlorid wird 15 Stunden lang am Rückfluss gekocht. Danach wird das Reaktionsgemisch wieder abgekühlt, und es wird Wasser dazugegeben. Die organische Phase wird abgetrennt, mit Wasser gewaschen, über wasserfreiem Magnesiumsulfat getrocknet und bei verringertem Druck eingedampft. Der Rückstand wird an einer Silikagelsäule mit Chloroform als Eluiermittel gereinigt. Die entsprechenden Fraktionen werden im Vakuum eingedampft, der Rückstand wird in Äthylalkohol gelöst und mit der äthanolischen Lösung von

Maleinsäure behandelt. Das Dimaleatsalz sondert sich kristallin ab, es wird filtriert und getrocknet. Schmelzpunkt: 192—194°C.

Elementaranalyse der Base, auf die Formel $C_{35}H_{36}Cl_4N_2O_2$ berechnet:
C = 63,84%; H = 5,51%; Cl = 21,54%; N = 4,25%;
gefunden: C = 63,62%; H = 5,67%; Cl = 21,70%; N = 4,28%.

Beispiel 6

1-[2'-⟨{4''-(Trifluormethyl)-phenyl}-{4'''-(fluor)-phenyl}-methoxy⟩-äthyl]-4-[3''''-⟨{4'''''-(trifluormethyl)-phenyl}-{4''''''-(fluor)-phenyl}-methoxy⟩-propyl]-piperazindimaleat

Die Suspension von 5,7 g 1-(2-Hydroxyäthyl)-4-(3-hydroxypropyl)pipeazin, 2,7 g 60%-iger öliger Natriumhydrid-dispersion und 50 ml wasserfreiem Xylol wird in Argonatmosphäre unter Rühren bis zum Aufhören der Wasserstoffentwicklung gekocht. Dann wird die mit 50 ml wasserfreiem Xylol bereitete Lösung von 22,3 g 4-Fluor-4'-(trifluormethyl)-benzhydrylbromid zugetropft und weitere 3 Stunden lang gekocht. Nach Abkühlen wird das Reaktionsgemisch auf Wasser gegossen, die organische Phase wird abgetrennt, mit Wasser gewaschen, über wasserfreiem Natriumsulfat getrocknet und bei verringertem Druck eingedampft. Der Rückstand wird in Äthanol gelöst, und nach Klären mit Knochenkohle wird die äthanolische Lösung von Maleinsäure zugegeben. Das Produkt wird filtriert und aus Methanol umkristallisiert. So erhält man die Titelverbindung mit einem Schmelzpunkt von 181—183°C.

Elementaranalyse der Base, auf die Formel $C_{37}H_{36}F_8N_2O_2$ berechnet:
C = 64,15%; H = 5,24%; F = 21,94%; N = 4,04%;
gefunden: C = 64,33%; H = 5,46%; F = 22,85%; N = 4,20%.

Beispiel 7

Aus den erfindungsgemässen Verbindungen können Arneimittelpräparate zum Beispiel der folgenden Zusammensetzung hergestellt werden:

Tabletten

50 g Wirkstoff, 92 g Lactose, 40 g Kartoffelstärke, 4 g Polyvinylpyrrolidon, 6 g Talk, 1 g Mangesiumstearat, 1 g kolloidales Siliziumdioxyd (Aerosil) und 6 g Ultraamylopectin werden miteinander vermischt, dann werden durch Nassgranulieren und Pressen Tabeletten von 200 mg, deren Wirkstoffgehalt jeweils 50 mg beträgt, hergestellt.

Wirkstoff: 1-[2'-⟨bis-{4''-(Chlor)-phenyl}-methoxy⟩-äthyl]-4-[3'''-⟨bis-{4''''-(chlor)-phenyl}-methoxy⟩-propyl]-piperazindimaleat.

Dragees

Die auf die oben beschriebene Weise hergestellten Tabletten werden in bekannter Weise mit einer aus Zucker und Talk bestehenden Schicht überzogen. Die Dragees werden mit einem Gemisch aus Bienenwachs und Carnaubawachs poliert. Gewicht der Dragees: 250 mg.

Suspension

Zusammensetzung von 100 ml Suspension:

| | |
|---|---|
| Wirkstoff | 1,0 g |
| Natriumhydroxyd | 0,26 g |
| Zitronensäure | 0,30 g |
| Nipagin (4-Hydroxybenzosäuremethylester) | 0,10 g |
| Carbopol® 940 (Polyacrylsäure) | 0,30 g |
| Äthanol (96%-ig) | 1,00 g |
| Himbeeraroma | 0,60 g |
| Sorbit (70%-ige wässrige Lösung) | 71,00 g |
| mit destilliertem Wasser auf | 100 ml aufgefüllt. |

In die mit 20 ml destilliertem Wasser bereitete Lösung von Nipagin und Zitronensäure wird in kleinen Portionen und unter intensivem Rühren das Carbopol® gegeben, dann wird die Lösung 10—12 Stunden stehengelassen. Danach werden die mit 1 ml destilliertem Wasser bereitete Lösung der obigen Menge Natriumhydroxyd, die wässrige Lösung von Sorbit und schliesslich unter Rühren die äthanolische Himbeeraroma-Lösung zugetropft. Zum Trägerstoff wird in kleinen Portionen der Wirkstoff gegeben, und

es wird mit einem eintauchenden Homogenisator suspendiert. Schliesslich wird die Suspension mit destilliertem Wasser auf 100 ml aufgefüllt, und der Suspensions-sirup wird durch eine Kolloidmühle gegeben.

Wirkstoff: 1-[2'-⟨bis-{4''-(Fluor)-phenyl}-methoxy⟩-äthyl]-4-[3'''-⟨bis-{4''''-(fluor)-phenyl}-methoxy⟩-propyl]-piperazindimaleat.

**Pätentansprüche für die Vertragsstaaten: BE CH DE FR GB IT LI LU NL SE**

1. Gegebenenfalls substituierte, 1-{2'-[bis-(Phenyl)-methoxy]-äthyl}-4-{3'-[bis-(phenyl)-methoxy]-propyl}-piperazine der allgemeinen Formel

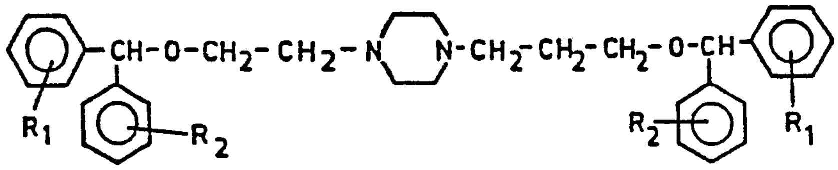

I,

worin

$R_1$ und $R_2$, die gleich oder verschieden sein können, für Wasserstoffatome, Halogenatome, Alkylreste mit 1 bis 4 Kohlenstoffatomen, Trihalogenmethylreste oder Alkoxyreste mit 1 bis 4 Kohlenstoffatomen stehen,

sowie ihre Säureadditionssalze und optisch aktiven Isomere und Solvate.

2. 1-{2'-[bis-(Phenyl)-methoxy]-äthyl}-4-{3'-[bis-(phenyl)-methoxy]-propyl}-piperazine nach Anspruch 1, dadurch gekennzeichnet, daß das beziehungsweise die Halogenatome(e), für welche[s] $R_1$ und/oder $R_2$ stehen kann beziehungsweise können, und/oder die Halogenatome des beziehungsweise der Trihalogenmethylreste[s], für welche[n] $R_1$ und/oder $R_2$ stehen kann beziehungsweise können, Fluor und/oder Chlor ist beziehungsweise sind.

3. 1-{2'-[bis-(Phenyl)-methoxy]-äthyl}-4-{3'-[bis-(phenyl)-methoxy]-propyl}-piperazine nach Anspruch 1 oder 2, dadurch gekennzeichnet, daß der beziehungsweise die niedere(n) Alkylrest(e), für welche[n] $R_1$ und/oder $R_2$ stehen kann beziehungsweise können, [ein] solche[r] mit 1 oder 2 Kohlenstoffatom(en) ist beziehungsweise sind.

4. 1-{2'-[bis-(Phenyl)-methoxy]-äthyl}-4-{3'-[bis-(phenyl)-methoxy]-propyl}-piperazine nach Anspruch 1 oder 2, dadurch gekennzeichnet, daß der beziehungsweise die niedere(n) Alkoxyrest(e), für welche[n] $R_1$ und/oder $R_2$ stehen kann beziehungsweise können, [ein] solche[r] mit 1 oder 2 Kohlenstoffatom(en) ist beziehungsweise sind.

5. 1-[2'-⟨bis-{4''-(Fluor)-phenyl}-methoxy⟩-äthyl]-4-[3'''-⟨bis-{4''''-(fluor)-phenyl}-methoxy⟩-propyl]-piperazin,

1-[2'-⟨{4''-(Methoxy)-phenyl}-{3'''-(trifluormethyl)-phenyl}-methoxy⟩-äthyl]-4-[3''''-⟨{4'''''-(methoxy)-phenyl}-{3''''''-(trifluormethyl)-phenyl}-methoxy⟩-propyl]-piperazin,

1-[2'-⟨bis-{4''-(Chlor)-phenyl}-methoxy⟩-äthyl]-4-[3'''-⟨bis-{4''''-(chlor)-phenyl}-methoxy⟩-propyl]-piperazin,

1-[2'-⟨{2''-(Methyl)-phenyl}-{4'''-(fluor)-phenyl}-methoxy⟩-äthyl]-4-[3''''-⟨{2'''''-(methyl)-phenyl}-{4''''''-(fluor)-phenyl}-methoxy⟩-propyl]-piperazin und 1-[2'-⟨Di-(phenyl)-methoxy⟩-äthyl]-4-[3''-⟨di-(phenyl)-methoxy⟩-propyl]-piperazin sowie ihre Säureadditionssalze.

6. Verfahren zur Herstellung der Verbindungen nach Anspruch 1 bis 5, dadurch gekennzeichnet, daß man,

a) gegebenenfalls substituierte, Benzhydrylhalogenide, Benzhydrole beziehungsweise ihre Alkoholate der allgemeinen Formel

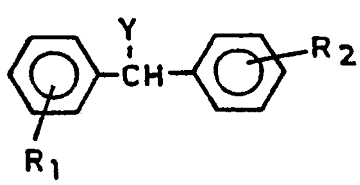

II,

worin

$R_1$ und $R_2$ die in den Ansprüchen 1 bis 4 angegebenen Bedeutungen haben und

Y für ein Halogenatom, eine Hydroxygruppe oder einen Rest der Formel

—OM,

in welchletzterer M ein Alkalimetallatom bedeutet, steht,

mit, gegebenenfalls substituierten, 1-[2'-(Halogen)-äthyl]-, 1-[2'-(Alkylsulfonyloxy)-äthyl]-, 1-[2'-(Arylsulfonyloxy)-äthyl]-, 1-[2'-(Hydroxy)-äthyl]- beziehungsweise 1-[2'-(Metalloxy)-äthyl]-4-[3''-(halogen)-propyl]-piperazinen, -4-[3''-(alkylsulfonyloxy)-propyl]-piperazinen, -4-[3''-(arylsulfonyloxy)-propyl]-piperazinen, -4-[3''-(hydroxy)-propyl]-piprazinen, beziehungsweise -4-[3''-(metalloxy)-propyl]-piperazinen der allgemeinen Formel

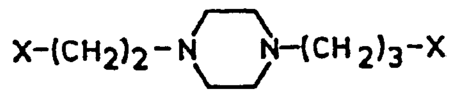 III,

worin

X für ein Halogenatom, einen Alkylsulfonyloxyrest, einen Arylsulfonyloxyrest, eine Hydroxygruppe oder einen Rest der allgemeinen Formel

—OM,

in welchletzterer M die obige Bedeutung hat, steht,

mit der Einschränkung, daß

α) X für eine Hydroxygruppe oder einen Rest der allgemeinen Formel —OM steht, wenn Y ein Halogenatom bedeutet, oder

β) X für ein Halogenatom, einen Alkylsulfonyloxyrest oder einen Arylsulfonyloxyrest steht, wenn Y eine Hydroxygruppe oder einen Rest der allgemeinen Formel —OM bedeutet,

umgesetzt werden, wobei man die Reaktion, wenn von den Substituenten Y und X der eine für eine Gruppe der Formel —OM und der andere für ein Halogenatom steht, in wasserfreiem Medium in einem neutralen organischen Lösungsmittel, nämlich einem aliphatischen oder aromatischen Kohlenwasserstoff, einem aliphatischen oder cycloaliphatischen Äther oder Hexamethyl-phosphoramid oder einem Gemisch derselben, durchgeführt wird oder, wenn das Alkoholat der Verbindung der allgemeinen Formel (II) oder (III) mit einem Alkalimetalltertiäralkoholat gebildet wird, der entsprechende tertiäre Alkohol oder auch sein mit den obigen Lösungsmitteln gebildetes Gemisch als Lösungsmittel dient, oder,

b) gegebenenfalls substituierte, 1-{ω-[bis-[Phenyl]-methoxy]-alkyl}-piperazine der allgemeinen Formel

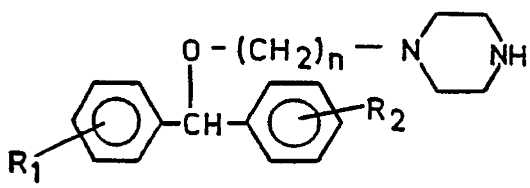 IV,

worin

$R_1$ und $R_2$ die in den Ansprüchen 1 bis 4 angegebenen Bedeutungen haben und

n für 2 oder 3 steht,

mit, gegebenenfalls substituierten, [Di-(phenyl)]-[ω-(halogen)-alkoxy]-methanen, [Di-(phenyl)]-[ω-(alkylsulfonyloxy)-alkoxy]-methanen beziehungsweise [Di-(phenyl)]-[ω-(arylsulfonyloxy)-alkoxy]-methanen der allgemeinen Formel

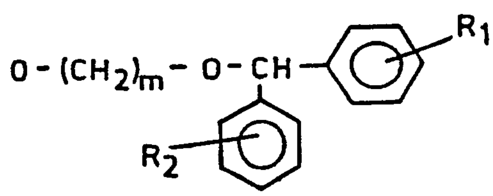 V

worin

$R_1$ und $R_2$ die in den Ansprüchen 1 bis 4 angegebenen Bedeutungen haben,

Q für ein Halogenatom, einen Alkylsulfonyloxyrest oder einen Arylsulfonyloxyrest steht und

m 2 oder 3 ist,

mit der Maßgabe, daß

m von n verschieden ist,

umsetzt, wobei die Reaktion des monosubstituierten Piperazines der allgemeinen Formel (IV) und der Verbindung der allgemeinen Formel (V) in einem inerten organischen Lösungsmittel, nämlich in einem aromatischen Kohlenwasserstoff, einem Äther, einem Keton, einem Säureamid, einem Ester, einem niederen Alkanol oder einem halogenierten Kohlenwasserstoff durchgeführt wird und eine Reaktionstemperatur zwischen 40°C und dem Siedepunkt des Reaktionsgemsiches einhält,

c) gegebenenfalls substituierte, Benzhydrylhalogenide, Benzhydrole beziehungsweise ihre Alkoholate der allgemeinen Formel II mit gegebenenfalls substituierten, 1-{ω-[bis-(Phenyl)-methoxy]-alkyl}-4-{ω-(halogen)-alkyl}-piperazinen, -4-{ω-(alkylsulfonyloxy)-alkyl}-piperazinen, -4-{ω-(arylsulfonyloxy)-alkyl}-

piperazinen, -4-{ω-(hydroxy)-alkyl}-piperazinen beziehungsweise -4-{ω-(metalloxy)-alkyl}-piperazinen der allgemeinen Formel

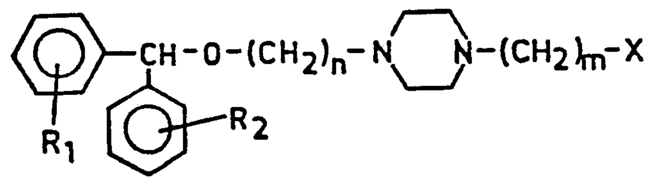 VI,

worin

$R_1$, $R_2$die in den Ansprüchen 1 bis 4 angegebenen Bedeutungen haben und

X, m und n die obigen Bedeutungen haben,

mit den Maßgaben, daß

α) X für eine Hydroxygruppe oder einen Rest der allgemeinen Formel —OM steht, wenn Y ein halogenatom bedeutet, oder

β) X für ein Halogenatom, einen Alkylsulfonyloxyrest oder einen Arylsulfonyloxyrest steht, wenn Y eine Hydroxygruppe oder einen Rest der allgemeinen Formel —OM bedeutet, sowie ferner

γ) m von n verschieden ist,

umsetzt, wobei die unter a) eingehaltenen Reaktionsbedingungen eingehalt, sowie in an sich bekannter Weise gegebenenfalls die nach einer der Varianten a) bis c) erhaltenen 1-{2'-[bis-(Phenyl)-methoxy]-äthyl}-4-{3'-[bis-(phenyl)-methoxy]-propyl}-piperazine der allgemeinen Formel I mit anorganischen oder organischen Säuren in Säureadditionssalze überführt und/oder die erhaltenen Säureadditionssalze der 1-{2'-[bis-(Phenyl)-methoxy]-äthyl}-4-{3'-[bis-(phenyl)-methoxy]-propyl}-piperazine der allgemeinen Formel I in die entsprechenden freien 1-{2'-bis-(Phenyl)-methoxy]-äthyl}-4-{3'-[bis-(phenyl)-methoxy]-propyl}-piperazinbasen der allgemeinen Formel I oder in andere Säureadditionssalze überführt und/oder gegebenenfalls eine Spaltung der erhaltenen racemischen 1-{2'-[bis-(Phenyl)-methoxy]-äthyl}-4-{3'-[bis-(phenyl)-methoxy]-propyl}-piperazine der allgemeinen Formel I beziehungsweise von deren Säureadditionssalzen beziehungsweise eine Racemisierung der entsprechenden optisch aktiven Verbindungen in Stereoisomere vornimmt.

7. Arzneimittel, gekennzeichnet durch einen Gehalt an 1 oder mehr Verbindung(en) nach Anspruch 1 bis 5 als Wirkstoff(en), gegebenenfalls zusammen mit 1 oder mehr üblichen pharmazeutischen Trägers- und/oder Hilfsstoff(en).

**Patentansprüche für den Vertragsstaat: AT**

1. Verfahren zur Herstellung von gegebenenfalls substituierten, 1-{2'-[bis-(Phenyl)-methoxy]-äthyl}-4-{3'-[bis-(phenyl)-methoxy]-propyl}-piperazinen der allgemeinen Formel

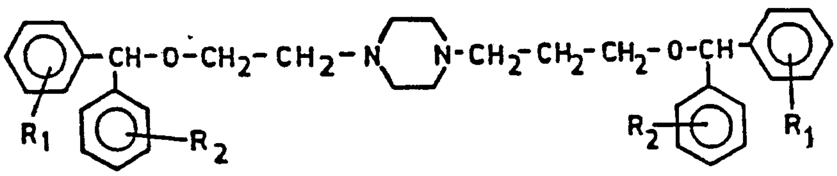 I,

worin

$R_1$ und $R_2$, die gleich oder verschieden sein können, für Wasserstoffatome, Halogenatome, Alkylreste mit 1 bis 4 Kohlenstoffatomen, Trihalogenmethylreste oder Alkoxyreste mit 1 bis 4 Kohlenstoffatomen stehen,

sowie ihrer Säureadditionssalze und optisch aktiven Isomeren und Solvate, dadurch gekennzeichnet, daß man

a) gegebenenfalls substituierte, Benzhydrylhalogenide, Benzhydrole beziehungsweise ihre Alkoholate der allgemeinen Formel

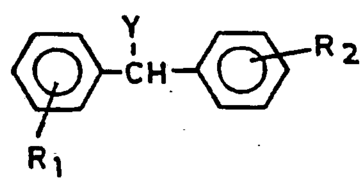 II,

worin

$R_1$ und $R_2$ die in den Ansprüchen 1 bis 4 angegebenen Bedeutungen haben und

Y für ein Halogenatom, eine Hydroxygruppe oder einen Rest der Formel

—OM,

in welchletzterer M ein Alkalimetallatom bedeutet, steht,

mit, gegebenenfalls substituierten, 1-[2'-(Halogen)-äthyl]-, 1-[2'-(Alkylsulfonyloxy)-äthyl]-, 1-[2'-(Arylsulfonyloxy)-äthyl]-, 1-[2'-(Hydroxy)-äthyl]- beziehungsweise 1-[2'-(Metalloxy)-äthyl]-4-[3''-(halogen)-propyl]-piperazinen, -4-[3''-(alkylsulfonyloxy)-propyl]-piperazinen, -4-[3''-(arylsulfonyloxy)-propyl]-piperazinen, -4-[3''-(hydroxy)-propyl]-piperazinen, beziehungsweise -4-[3''-(metalloxy)-propyl]-piperazinen der allgemeinen Formel

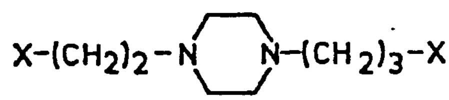

III,

worin

X für ein Halogenatom, einen Alkylsulfonyloxyrest, einen Arylsulfonyloxyrest, eine Hydroxygruppe oder einen Rest der allgemeinen Formel

—OM,

in welchletzterer M die obige Bedeutung hat, steht,

mit der Einschränkung, daß

α) X für eine Hydroxygruppe oder einen Rest der allgemeinen Formel —OM steht, wenn Y ein Halogenatom bedeutet, oder

β) X für ein Halogenatom, einen Alkylsulfonyloxyrest oder einen Arylsulfonyloxyrest steht, wenn Y eine Hydroxygruppe oder einen Rest der allgemeinen Formel —OM bedeutet,

umgesetzt werden, wobei die Reaktion, wenn von den Substituenten Y und X der eine für eine Gruppe der Formel —OM und der andere für ein Halogenatom steht, in wasserfreiem Medium in einem neutralen organischen Lösungsmittel, nämlich einem aliphatischen oder aromatischen Kohlenwasserstoff, einem aliphatischen oder cycloaliphatischen Äther oder Hexamethyl-phosphoramid oder einem Gemisch derselben, durchgeführt wird oder, wenn das Alkoholat der Verbindung der allgemeinen Formel (II) oder (III) mit einem Alkalimetalltertiäralkoholat gebildet wird, der entsprechende tertiäre Alkohol oder auch sein mit den obigen Lösungsmitteln gebildetes Gemisch als Lösungsmittel dient, oder,

b) gegebenenfalls substituierte, 1-{ω-[bis-[Phenyl]-methoxy]-alkyl}-piperazine der allgemeinen Formel

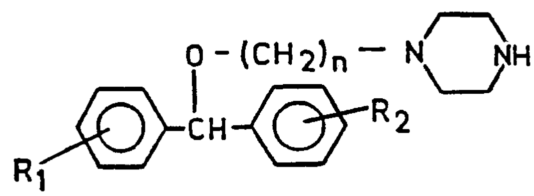

IV,

worin

$R_1$ und $R_2$ die in den Ansprüchen 1 bis 4 angegebenen Bedeutungen haben und

n für 2 oder 3 steht,

mit, gegebenenfalls substituierten, [Di-(phenyl)]-[ω-(halogen)-alkoxy]-methanen, [Di-(phenyl)]-[ω-(alkylsulfonyloxy)-alkoxy]-methanen beziehungsweise [Di-(phenyl)]-[ω-(arylsulfonyloxy)-alkoxy]-methanen der allgemeinen Formel

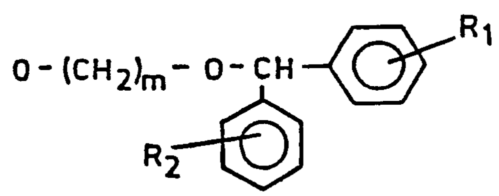

V

worin

$R_1$ und $R_2$ die in den Ansprüchen 1 bis 4 angegebenen Bedeutungen haben,

Q für ein Halogenatom, einen Alkylsulfonyloxyrest oder einen Arylsulfonyloxyrest steht und

m 2 oder 3 ist,

mit der Maßgabe, daß

m von n verschieden ist,

umsetzt, wobei die Reaktion des monosubstituierten Piperazines der allgemeinen Formel (IV) und der Verbindung der allgemeinen Formel (V) in einem inerten organischen Lösungsmittel, nämlich in einem aromatischen Kohlenwasserstoff, einem Äther, einem Keton, einem Säureamid, einem Ester, einem niederen Alkanol oder einem halogenierten Kohlenwasserstoff durchgeführt wird und eine Reaktionstemperatur zwischen 40°C und dem Siedepunkt des Reaktionsgemsiches einhält, oder,

c) gegebenenfalls substituierte, Benzhydrylhalogenide, Benzhydrole beziehungsweise ihre Alkoholate der allgemeinen Formel II mit gegebenenfalls substituierten, 1-{ω-[bis-(Phenyl)-methoxy]-alkyl}-4-{ω-(halogen)-alkyl}-piperazinen, -4-{ω-(alkylsulfonyloxy)-alkyl}-piperazinen, -4-{ω-(arylsulfonyloxy)-alkyl}-

piperazinen, -4-{ω-(hydroxy)-alkyl}-piperazinen beziehungsweise -4-{ω-(metalloxy)-alkyl}-piperazinen der allgemeinen Formel

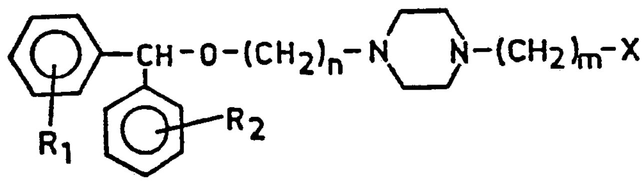 VI,

worin

$R_1$, $R_2$ die in den Ansprüchen 1 bis 4 angegebenen Bedeutungen haben und

X, m und n die obigen Bedeutungen haben,

mit den Maßgaben, daß

α) X für eine Hydroxygruppe oder einen Rest der allgemeinen Formel —OM steht, wenn Y ein halogenatom bedeutet, oder

β) X für ein Halogenatom, einen Alkylsulfonyloxyrest oder einen Arylsulfonyloxyrest steht, wenn Y eine Hydroxygruppe oder einen Rest der allgemeinen Formel —OM bedeutet, sowie ferner

γ) m von n verschieden ist,

umsetzt, wobei die unter a) eingehaltenen Reaktionsbedingungen eingehalt, sowie in an sich bekannter Weise gegebenenfalls die nach einer der Varianten a) bis c) erhaltenen 1-{2'-[bis-(Phenyl)-methoxy]-äthyl}-4-{3'-[bis-(phenyl)-methoxy]-propyl}-piperazine der allgemeinen Formel I mit anorganischen oder organischen Säuren in Säureadditionssalze überführt und/oder die erhaltenen Säureadditionssalze der 1-{2'-[bis-(Phenyl)-methoxy]-äthyl}-4-{3'-[bis-(phenyl)-methoxy]-propyl}-piperazine der allgemeinen Formel I in die entsprechenden freien 1-{2'-bis-(Phenyl)-methoxy]-äthyl}-4-{3'-[bis-(phenyl)-methoxy]-propyl}-piperazinbasen der allgemeinen Formel I oder in andere Säureadditionssalze überführt und/oder gegebenenfalls eine Spaltung der erhaltenen racemischen 1-{2'-[bis-(Phenyl)-methoxy]-äthyl}-4-{3'-[bis-(phenyl)-methoxy]-propyl}-piperazine der allgemeinen Formel I beziehungsweise von deren Säureadditionssalzen beziehungsweise eine Racemisierung der entsprechenden optisch aktiven Verbindungen in Stereoisomere vornimmt.

2. Verfahren nach Anspruch 1, dadurch gekennzeichnet, daß solche gegebenenfalls substituierte 1-{2'-[bis-(Phenyl)-methoxy]-äthyl}-4-{3'-[bis-(phenyl)-methoxy]-propyl}-piperazine hergestellt werden, bei denen das beziehungsweise die Halogenatome(e), für welche[s] $R_1$ und/oder $R_2$ stehen kann beziehungsweise können, und/oder die Halogenatome des beziehungsweise der Trihalogenmethylreste[s], für welche[n] $R_1$ und/oder $R_2$ stehen kann beziehungsweise können, Fluor und/oder Chlor ist beziehungsweise sind.

3. Verfahren nach Anspruch 1 oder 2, dadurch gekennzeichnet, daß solche gegebenenfalls substituierte 1-{2'-[bis-(Phenyl)-methoxy]-äthyl}-4-{3'-[bis-(phenyl)-methoxy]-propyl}-piperazine hergestellt werden, bei denen der beziehungsweise die niedere(n) Alkylrest(e), für welche[n] $R_1$ und/oder $R_2$ stehen kann beziehungsweise können, [ein] solche[r] mit 1 oder 2 Kohlenstoffatom(en) ist beziehungsweise sind.

4. Verfahren nach Anspruch 1 oder 2, dadurch gekennzeichnet, daß solche gegebenenfalls substituierte 1-{2'-[bis-(Phenyl)-methoxy]-äthyl}-4-{3'-[bis-(phenyl)-methoxy]-propyl}-piperazine hergestellt werden bei denen der beziehungsweise die niedere(n) Alkoxyrest(e), für welche[n] $R_1$ und/oder $R_2$ stehen kann beziehungsweise können, [ein] solche[r] mit 1 oder 2 Kohlenstoffatom(en) ist beziehungsweise sind.

5. Verfahren nach Anspruch 1, dadurch gekennzeichnet, daß

1-[2'-⟨bis-{4''-(Fluor)-phenyl}-methoxy⟩-äthyl]-4-[3'''-⟨bis-{4''''-(fluor)-phenyl}-methoxy⟩-propyl]-piperazin,

1-[2'-⟨{4''-(Methoxy)-phenyl}-{3'''-(trifluormethyl)-phenyl}-methoxy⟩-äthyl]-4-[3''''-⟨{4'''''-(methoxy)-phenyl}-{3''''''-(trifluormethyl)-phenyl}-methoxy⟩-propyl]-piperazin,

1-[2'-⟨bis-{4''-(Chlor)-phenyl}-methoxy⟩-äthyl]-4-[3'''-⟨bis-{4''''-(chlor)-phenyl}-methoxy⟩-propyl]-piperazin,

1-[2'-⟨{2''-(Methyl)-phenyl}-{4'''-(fluor)-phenyl}-methoxy⟩-äthyl]-4-[3''''-⟨{2'''''-(methyl)-phenyl}-{4''''''-(fluor)-phenyl}-methoxy⟩-propyl]-piperazin und 1-[2'-⟨Di-(phenyl)-methoxy⟩-äthyl]-4-[3''-⟨di-(phenyl)-methoxy⟩-propyl]-piperazin sowie ihre Säureadditionssalze hergestellt werden.

**Revendications pour les Etats contractants: BE CH DE FR GB IT LI LU NL SE**

1. 1-{2′-[bis-(phényl)-méthoxy]-éthyl}-4-{3′-[bis-(phényl)-méthoxy]-propyl}-pipérazines éventuellement substituées, répondant à la formule générale

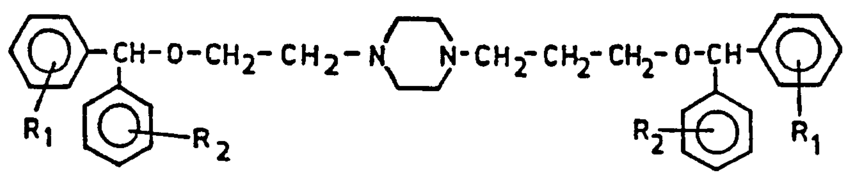

I,

dans laquelle

$R_1$ et $R_2$ qui peuvent être identiques ou différents, représentent des atomes d'hydrogène, des atomes d'halogène, des restes alkyle comportant 1 à 4 atomes de carbone, des restes de trihalogénométhyle ou des restes alkoxy comportant 1 à 4 atomes de carbone, ainsi que leurs sels formés par addition d'acides et des isomères et des solvates actifs optiquement.

2. 1-{2′-[bis-(phényl)-méthoxy]-éthyl}-4-{3′-[bis-(phényl)-méthoxy]-propyl}-pipérazines selon la revendication 1, caractérisé en ce que le ou les atomes d'halogène, que peuvent représenter $R_1$ et/ou $R_2$, et/ou les atomes d'halogène du ou des restes de trihalogénométhyle, que peuvent représenter $R_1$ et/ou $R_2$, est ou sont le fluor et/ou le chlore.

3. 1-{2′-[bis-(phényl)-méthoxy]-éthyl}-4-{3′-[bis-(phényl)-méthoxy]-propyl}-pipérazines selon la revendication 1 ou 2, caractérisé en ce que le ou les faibles restes alkyle, que peuvent représenter $R_1$ et/ou $R_2$, est ou sont un ou des restes alkyle comportant 1 ou deux atomes de carbone.

4. 1-{2′-[bis-(phényl)-méthoxy]-éthyl}-4-{3′-[bis-(phényl)-méthoxy]-propyl}-pipérazines selon la revendication 1 ou 2, caractérisé en ce que le ou les faibles restes alkyle, que peuvent représenter $R_1$ et/ou $R_2$, est ou sont un ou des restes alkoxy comportant 1 ou deux atomes de carbone.

5. 1-{2′-<bis-{4″-(fluor)-(phényl}-méthoxy>-éthyl]-4-[3‴-<bis-{4⁗-(fluor)-phényl}-méthoxy>-propyl]-pipérazine,

1-{[2′-<{4″-(méthoxy)-phényl}-{3‴-(trifluorométhyl)-phényl}-méthoxy>-éthyl]-4-[3⁗-{{4′′′′′-(méthoxy)-phényl}-{3′′′′′′-(trifluorométhyl)-phényl}-méthoxy>-propyl]-pipérazine,

1-[2′-<bis-{4″-(chlor)-phényl)}-méthoxy>-éthyl]-4-[3‴-<bis-{4⁗-chlore)-phényl}-méthoxy>-propyl]-pipérazine,

1-[2′-<{2″-(méthyl)-phényl}-{4‴-(fluor)-phényl}-méthoxy>-éthyl]-4-[3⁗-<{2′′′′′-(méthyl)-phényl}-{4′′′′′′-(fluor)-phényl}-méthoxy>-propyl]-pipérazine et 1-[2′-<di(phényl)-méthoxy>-éthyl]-4-[3″-<di-(phényl)-méthoxy>-propyl]-pipérazine ainsi que leurs sels formés par addition d'acides.

6. Procédé pour préparer les composés selon les revendications 1 à 5, caractérisé en ce que

a) on fait réagir des halogénures de benzhydryle, des benzhydroles ou leurs alcoolates répondant à la formule générale

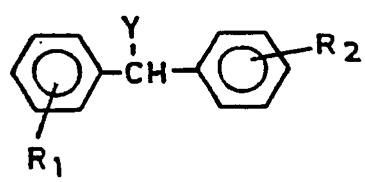

II,

$R_1$ et $R_2$ ayant les significations indiquées dans les revendications 1 à 4, et

Y représentant un atome d'halogène, un groupe hydroxy ou un reste répondant à la formule —OM, dans laquelle M est un atome d'un métal alcalin,

avec des 1-[2′-(halogène)-éthyl]-, des 1-[2′-(alkylsulfonyloxy)-éthyl]-, des 1-[2′-(arysulfonyloxy)éthyl]-, des 1-2′-(hydroxy)-éthyl]- ou des 1-[2′-(métaloxy)-éthyl]-4-(3″-(halogène)-propyl]-pipérazines, des -4-[3″-(alkylsulfonyloxy)-propyl]-pipérazines, des -4-[3″-(arylsulfonyloxy)-propyl]-pipérazine, des -4-[3″-(hydroxy)-propyl]-pipérazines ou des -4-[3″-(métaloxy)-propyl]-pipérazines, éventuellement substituées, répondant à la formule générale

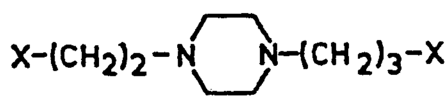

III,

X étant un atome d'halogène, un reste alkylsulfonyloxy, un reste arylsulfonyloxy, un groupe hydroxy ou un reste répondant à la formule générale —OM, dans laquelle M possède la signification indiquée plus haut, avec la condition selon laquelle

α) X représente un groupe hydroxy ou un reste répondant à la formule générale —OM, lorsque Y désigne un atome d'halogène, ou

β) X représente un atome d'halogène, un reste alkylsulfonyloxy ou un reste arylsulfonyloxy, lorsque Y désigne un groupe hydroxy ou un reste répondant à la formule générale —OM,

auquel cas, lorsque l'un des substituants Y et X représente un groupe répondant à la formule —OM et que l'autre représente un atome d'halogène, la réaction est exécutée dans un milieu exempt d'eau, dans un solvant organique neutre, à savoir un hydrocarbure aliphatique ou aromatique, un éther aliphatique ou cycloaliphatique ou de l'hexaméthylphosphoramide ou un mélange de telles substances ou bien, lorsque l'alcoolate du composé répondant à la formule générale (I) ou (III) est formé avec un alcoolate tertiaire d'un métal alcalin, l'alcoolate tertiaire correspondant ou même son mélange formé avec les solvants indiqués plus haut, est utilisé comme solvant, ou

b) on fait réagir des 1-{ω-[bis[phényl]-méthoxy]-alkyl}-pipérazines, éventuellement substituées, répondant à la formule générale

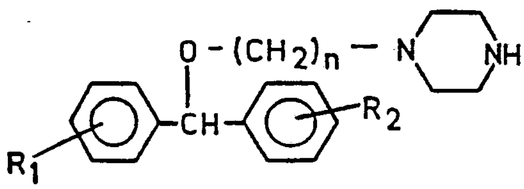

IV,

$R_1$ et $R_2$ ayant les significations indiquées dans les revendications 1 à 4 et
n étant égal à 2 ou 3,

avec des [di-(phényl)]-[ω-(halogène)-alkoxy]-méthanes, des [di-(phényl)]-[ω-(alkylsulfonyloxy)-alkoxy]-méthanes ou des [di-(phényl)]-[ω-(arylsulfonyloxy)-alkoxy]-méthanes, éventuellement substitués, répondant à la formule générale

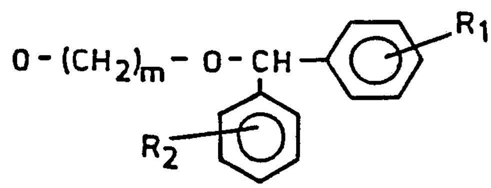

V

$R_1$ et $R_2$ ayant les significations indiquées dans la revendication 1 à 4,
Q représentant un atome d'halogène, un reste d'alkylsulfonyloxy ou un reste d'arylsulfonyloxy, et
m étant égal à 2 ou 3,
avec la condition que m est différent de n,

la réaction des pipérazines monosubstituées répondant à la formule générale (IV) et du composé répondant à la formule générale (V) étant exécutée dans un solvant organique inerte, à savoir dans un hydrocarbure aromatique, un éther, une cétone, une amide acide, un ester, un alkanol faible ou un hydrocarbure halogéné, tout en respectant une température de réaction entre 40°C et le point d'ébullition du mélange réactionnel,

c) on fait réagir des halogénures de benzhydriles, des benzhydroles, éventuellement substitués, ou leurs alcoolates répondant à la formule générale (II) avec des 1-{ω-[bis-(phényl)-méthoxy]-alkyl}-4-{ω-(halogène)-alkyl}-pipérazines, des -4-{ω-(alkylsulfonyloxy)-alkyl}-pipérazines, des -4-{ω-(arylsulfonyloxy)-alkyl}-pipérazines, des -4-{ω-(hydroxy)-alkyl}-pipérazines ou des -4-{ω-(métaloxy)-alkyl}-pipérazines, éventuellement substituées, répondant à la formule générale

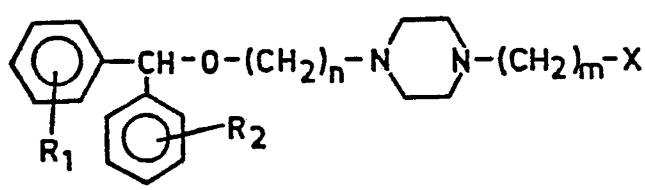

VI,

$R_1$ et $R_2$ ayant les significations indiquées dans les revendications 1 et 4, et
X, m et n possédant des significations indiquées plus haut,

avec les conditions selon lesquelles

α) X représente un groupe hydroxy ou un reste répondant à la formule —OM, lorsque Y désigne un atome d'halogène, ou

β) X représente un atome d'halogène, un reste alkylsulfonyloxy ou un reste arylsulfonyloxy, lorsque Y est un groupe hydroxy ou un reste répondant à la formule générale —OM, et en outre

γ) m est différent de n,

en respectant les conditions de réaction indiquées en a) et en transformant éventuellement, de façon connue en soi, les 1-{2'-[bis-(phényl)-méthoxy]-éthyl}-4-{3'-[bis-(phényl)-méthoxy]-propyl}-pipérazines, obtenues conformément à l'une des variantes a) à c) et répondant à la formule générale I avec des acides minéraux ou organiques pour obtenir des sels formés par addition d'acides, et/ou en transformant les sels obtenus formés par addition d'acides des 1-{2'-[bis-(phényl)-méthoxy]-éthyl}-4-{3'-[bis-(phényl)-méth-

oxy]-propyl}-pipérazines répondant à la formule générale I en les bases correspondantes libres de 1-{2'-[bis-(phényl)-méthoxy]-éthyl}-4-{3'-[bis-(phényl)-méthoxy]-propyl}-pipérazines répondant à la formule générale I ou en d'autres sels formés par addition d'acides, et/ou en réalisant éventuellement un fractionnement des 1-{2'-[bis-(phényl)-méthoxy]-éthyl}-4-{3-[bis-(phényl)-méthoxy]-propyl}-pipérazines reacémiques obtenues, répondant à la formule générale I ou des sels de ces pipérazines, formés par addition d'acides ou une racémisation des composés correspondantes, actifs du point de vue obtique, en des stéréoisomères.

7. Médicament, caractérisé en ce qu'il contient 1 ou plusieurs composés selon les revendications 1 à 5 en tant que substances actives, éventuellement conjointement avec 1 ou plusieurs substances porteuses et/ou auxiliaires pharmaceutiques usuelles.

**Revendications pour l'Etat contractant: AT**

1. Procédé pour préparer des composés 1-{2'-[bis-(phényl)-méthoxy]-éthyl}-4-{3'-[bis-(phényl)-méthoxy]-propyl}-pipérazines éventuellement substituées, répondant à la formule générale

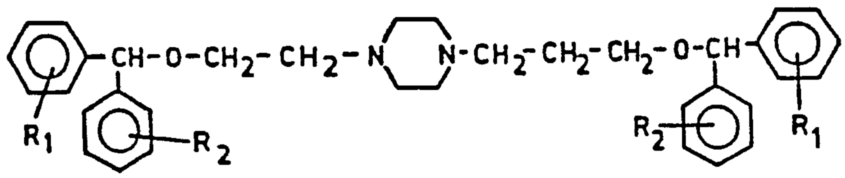

I,

dans laquelle

$R_1$ et $R_2$ qui peuvent étre identiques ou différents, représentent des atomes d'hydrogène, des atomes d'halogène, des restes alkyle comportant 1 à 4 atomes de carbone, des restes de trihalogénométhyle ou des restes alkoxy comportant 1 à 4 atomes de carbone, ainsi que leurs sels formés par addition d'acides et des isomères et des solvates actifs optiquement, caractérisé en ce que

a) on fait réagir des halogénures de benzhydryle, des benzhydroles ou leurs alcoolates répondant à la formule générale

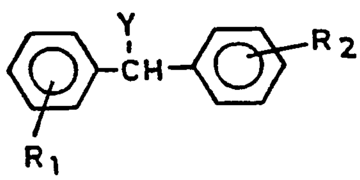

II,

$R_1$ et $R_2$ ayant les significations indiquées dans les revendications 1 à 4, et

Y représentant un atome d'halogène, un groupe hydroxy ou un reste répondant à la formule —OM, dans laquelle M est un atome d'un métal alcalin,

avec des 1-[2'-(halogène)-éthyl]-, des 1-[2'-(alkylsulfonyloxy)-éthyl]-, des 1-[2'-(arylsulfonyloxy)éthyl]-, des 1-2'-(hydroxy)-éthyl]- ou des 1-[2'-(métaloxy)-éthyl]-4-(3''-(halogène)-propyl]-pipérazines, des -4-[3''-(alkylsulfonyloxy)-propyl]-pipérazines, des -4-[3''-(arylsulfonyloxy)-propyl]-pipérazine, des -4-[3''-(hydroxy)-propyl]-pipérazines ou des -4-[3''-(métaloxy)-propyl]-pipérazines, éventuellement substituées, répondant à la formule générale

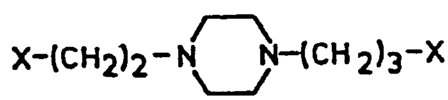

III,

X étant un atome d'halogène, un reste alkylsulfonyloxy, un reste arylsulfonyloxy, un groupe hydroxy ou un reste répondant à la formule générale —OM, dans laquelle M possède la signification indiquée plus haut, avec la condition selon laquelle

α) X représente un groupe hydroxy ou un reste répondant à la formule générale —OM, lorsque Y désigne un atome d'halogène, ou

β) X représente un atome d'halogène, un reste alkylsulfonyloxy ou un reste arylsulfonyloxy, lorsque Y désigne un groupe hydroxy ou un reste répondant à la formule générale —OM,

auquel cas, lorsque l'un des substituants Y et X représente un groupe répondant à la formule —OM et que l'autre représente un atome d'halogène, la réaction est exécutée dans un milieu exempt d'eau, dans un solvant organique neutre, à savoir un hydrocarbure aliphatique ou aromatique, un éther aliphatique ou cycloaliphatique ou de l'hexaméthylphosphoramide ou un mélange de telles substances ou bien, lorsque l'alcoolate du composé répondant à la formule générale (I) ou (III) est formé avec un alcoolate tertiaire d'un métal alcalin, l'alcoolate tertiaire correspondant ou même son mélange formé avec les solvants indiqués plus haut, est utilisé comme solvant, ou

b) on fait réagir des 1-{ω-[bis[phényl]-méthoxy]-alkyl}-pipérazines, éventuellement substituées, répondant à la formule générale

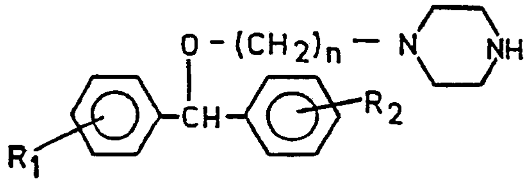

IV,

$R_1$ et $R_2$ ayant les significations indiquées dans les revendications 1 à 4 et
n étant égal à 2 ou 3,

avec des [di-(phényl)]-[ω-(halogène)-alkoxy]-méthanes, des [di-(phényl)]-[ω-(alkylsulfonyloxy)-alkoxy]-méthanes ou des [di-(phényl)]-[ω-(arylsulfonyloxy)-alkoxy]-méthanes, éventuellement substitués, répondant à la formule générale

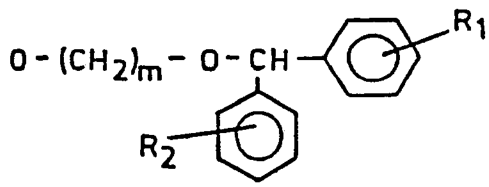

V

$R_1$ et $R_2$ ayant les significations indiquées dans la revendication 1 à 4,
Q représentant un atome d'halogène, un reste d'alkylsulfonyloxy ou un reste d'arylsulfonyloxy, et
m étant égal à 2 ou 3,
avec la condition que m est différent de n,

la réaction des pipérazines monosubstituées répondant à la formule générale (IV) et du composé répondant à la formule générale (V) étant exécutée dans un solvant organique inerte, à savoir dans un hydrocarbure aromatique, un éther, une cétone, une amide acide, un ester, un alkanol faible ou un hydrocarbure halogéné, tout en respectant une température de réaction entre 40°C et le point d'ébullition du mélange réactionnel,

c) on fait réagir des halogénures de benzhydriles, des benzhydroles, éventuellement substitués, ou leurs alcoolates répondant à la formule générale (II) avec des 1-{ω-[bis-(phényl)-méthoxy]-alkyl}-4-{ω-(halogène)-alkyl}-pipérazines, des -4-{ω-(alkylsulfonyloxy)-alkyl}-pipérazines, des -4-{ω-(arylsulfonyloxy)-alkyl}-pipérazines, des -4-{ω-(hydroxy)-alkyl}-pipérazines ou des -4-{ω-(métaloxy)-alkyl}-pipérazines, éventuellement substituées, répondant à la formule générale

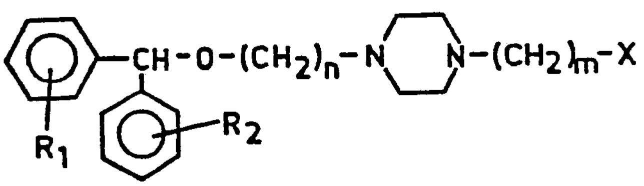

VI,

$R_1$ et $R_2$ ayant les significations indiquées dans les revendications 1 et 4, et
X, m et n possédant des significations indiquées plus haut,

avec les conditions selon lesquelles

α) X représente un groupe hydroxy ou un reste répondant à la formule —OM, lorsque Y désigne un atome d'halogène, ou

β) X représente un atome d'halogène, un reste alkylsulfonyloxy ou un reste arylsulfonyloxy, lorsque Y est un groupe hydroxy ou un reste répondant à la formule générale —OM, et en outre

γ) m est différent de n,

en respectant les conditions de réaction indiquées en a) et en transformant éventuellement, de façon connue en soi, les 1-{2'-[bis-(phényl)-méthoxy]-éthyl}-4-{3'-[bis-(phényl)-méthoxy]-propyl}-pipérazines, obtenues conformément à l'une des variantes a) à c) et répondant à la formule générale I avec des acides minéraux ou organiques pour obtenir des sels formés par addition d'acides, et/ou en transformant les sels obtenus formés par addition d'acides des 1-{2'-[bis-(phényl)-méthoxy]-éthyl}-4-{3'-[bis-(phényl)-méthoxy]-propyl}-pipérazines répondant à la formule générale I en les bases correspondantes libres de 1-{2'-[bis-(phényl)-méthoxy]-éthyl}-4-{3'-[bis-(phényl)-méthoxy]-propyl}-pipérazines répondant à la formule générale I ou en d'autres sels formés par addition d'acides, et/ou en réalisant éventuellement un fractionnement des 1-{2'-[bis-(phényl)-méthoxy]-éthyl}-4-{3-[bis-(phényl)-méthoxy]-propyl}-pipérazines reacémiques obtenues, répondant à la formule générale I ou des sels de ces pipérazines, formés par addition d'acides ou une racémisation des composés correspondantes, actifs du point de vue obtique, en des stéréoisomères.

2. Procédé selon la revendication 1 caractérisé en ce que 1-{2'-[bis-(phényl)-méthoxy]-éthyl}-4-{3'-[bis-(phényl)-méthoxy]-propyl}-pipérazines éventuellement substituées sont préparées en ce que le ou les atomes d'halogène, que peuvent représenter $R_1$ et/ou $R_2$, et/ou les atomes d'halogène du ou des restes de trihalogénométhyle, que peuvent représenter $R_1$ et/ou $R_2$, est ou sont le fluor et/ou le chlore.

3. Procédé selon la revendication 1 ou 2, caractérisé en ce que 1-{2'-[bis-(phényl)-méthoxy]-éthyl}-4-{3'-[bis-(phényl)-méthoxy]-propyl}-pipérazines éventuellement substituées sont preparées en ce que le ou les faibles restes alkyle, que peuvent représenter $R_1$ et/ou $R_2$, est ou sont un ou des restes alkyle comportant 1 ou deux atomes de carbone.

4. Procédé selon la revendication 1 ou 2, caractérisé en ce que 1-{2'-[bis-(phényl)-méthoxy]-éthyl}-4-{3'-[bis-(phényl)-méthoxy]-propyl}-pipérazines éventuellement substituées sont préparées en ce que le ou les faibles restes alkyle, que peuvent représenter $R_1$ et/ou $R_2$, est ou sont un ou des restes alkoxy comportant 1 ou deux atomes de carbone.

5. Procédé selon la revendication 1 caractérisé en ce que 1-[2'-<bis-{4''-(fluor)-(phényl}-méthoxy>-éthyl]-4-[3'''-<bis-{4''''-(fluor)-phényl}-méthoxy>-propyl]-pipérazine,

1-[2'-<{4''-(méthoxy)-phényl}-{3'''-(trifluorométhyl)-phényl}-méthoxy>-éthyl]-4-[3''''-<{4'''''-(méthoxy)-phényl}-{3''''''-(trifluorométhyl)-phényl}-méthoxy>-propyl]-pipérazine,

1-[2'-<bis-{4''-(chlor)-phényl)}-méthoxy>-éthyl]-4-[3'''-<bis-{4''''-chlore)-phényl}-méthoxy>-propyl]-pipérazine,

1-[2'-<{2''-(méthyl)-phényl}-{4'''-(fluor)-phényl}-méthoxy>-éthyl]-4-[3''''-<{2'''''-(méthyl)-phényl}-{4''''''-(fluor)-phényl}-méthoxy>-propyl]-pipérazine et 1-[2'-<di(phényl)-méthoxy>-éthyl]-4-[3''-<di-(phényl)-méthoxy>-propyl]-pipérazine ainsi que leurs sels formés par addition d'acides sont preparées.

**Claims for the Contracting States: BE CH DE FR GB IT LI LU NL SE**

1. Optionally substituted 1-{2'-[bis-(phenyl)-methoxy]-ethyl}-4-{3'-[bis-(phenyl)-methoxy]-propyl}-piperazines of the general formula

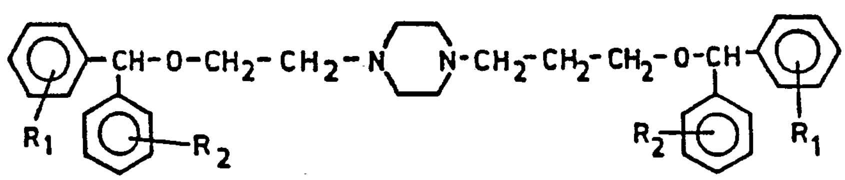

I,

wherein

$R_1$ and $R_2$, which may be identical or different, represent hydrogen atoms, halogen atoms, alkyl radicals having from 1 to 4 carbon atoms, trihalomethyl radicals or alkoxy radicals having from 1 to 4 carbon atoms, and their acid addition salts and optically active isomers and solvates.

2. 1-{2'-[bis-(phenyl)-methoxy]-ethyl}-4-{3'-[bis-(phenyl)-methoxy]-propyl}-piperazines according to claim 1, characterised in that the halogen atom(s) which $R_1$ and/or $R_2$ may represent and/or the halogen atoms of the trihalomethyl radical(s) which $R_1$ and/or $R_2$ may represent is(are) fluorine and/or chlorine.

3. 1-{2'-[bis-(phenyl)-methoxy]-ethyl}-4-{3'-[bis-(phenyl)-methoxy]-propyl}-piperazines according to claim 1 or 2, characterised in that the lower alkyl radical(s) which $R_1$ and/or $R_2$ may represent is(are) such (a) radical(s) containing 1 or 2 carbon atoms.

4. 1-{2'-[bis-(phenyl)-methoxy]-ethyl}-4-{3'-[bis-(phenyl)-methoxy]-propyl}-piperazines according to claim 1 or 2, characterised in that the lower alkoxy radical(s) which $R_1$ and/or $R_2$ may represent is(are) such (a) radical(s) containing 1 or 2 carbon atoms.

5. 1-[2'-<bis-{4''-(fluoro)-(phenyl}-methoxy>-ethyl]-4-[3'''-<bis-{4''''-(fluoro)-phenyl}-methoxy>-propyl]-piperazine,

1-[2'-<{4''-(methoxy)-phenyl}-{3'''-(trifluoromethyl)-phenyl}-methoxy>-ethyl]-4-[3''''-<{4'''''-(methoxy)-phenyl}-{3''''''-(trifluoromethyl)-phenyl}-methoxy>-propyl]-piperazine,

1-[2'-<bis-{4''-(chloro)-phenyl}-methoxy>-ethyl]-4-[3'''-<bis-{4''''-chloro)-phenyl}-methoxy>-propyl]-piperazine,

1-[2'-<{2''-(methyl)-phenyl}-{4'''-(fluoro)-phenyl}-methoxy>-ethyl]-4-[3''''-<{2'''''-(methyl)-phenyl}-{4''''''-(fluoro)-phenyl}-methoxy>-propyl]-piperazine and 1-[2'-<di(phenyl)-methoxy>-ethyl]-4-[3''-<di-(phenyl)-methoxy>-propyl]-piperazine, and their acid addition salts.

6. A process for the preparation of the compounds according to claims 1 to 5, characterised in that

a) optionally substituted benzhydryl halides, benzhydrols or alcoholates thereof of the general formula

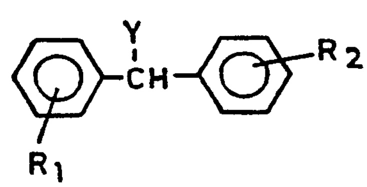

II,

wherein

$R_1$ and $R_2$, have the meanings given in claims 1 to 4, and

Y is a halogen atom, a hydroxy group or a radical of the formula —OM, in which M is an alkali metal atom, are reacted with optionally substituted 1-[2'-(halo)-ethyl]-, 1-[2'-(alkylsulphonyloxy)-ethyl]-, 1-[2'-(arylsulphonyloxy)-ethyl]-, 1-[2'-(hydroxy)-ethyl]- or 1-[2'-(metaloxy)-ethyl]-4-(3''-(halo)-propyl]-piperazines, -4-[3''-(alkylsulphonyloxy)-propyl]-piperazines, -4-[3''-(arylsulphonyloxy)-propyl]-piperazines, -4-[3''-(hydroxy)-propyl]-piperazines or -4-[3''-(metaloxy)-propyl]-piperazines of the general formula

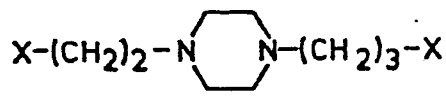

III,

wherein

X is a halogen atom, an alkylsulphonyloxy radical, an arylsulphonyloxy radical, a hydroxy group or a radical of the general formula —OM, wherein

M has the meaning given above, with the proviso that

α) X is a hydroxy group or a radical of the general formula —OM where Y is a halogen atom, or

β) X is a halogen atom, an alkylsulphonyloxy radical or an arylsulphonyloxy radical when Y is a hydroxy group or a radical of the general formula —OM,

and when one of the substituents Y and X is a group of the formula —OM and the other is a halogen atom, the reaction is carried out in an anhydrous medium in a neutral organic solvent, namely an aliphatic or aromatic hydrocarbon, an aliphatic or cycloaliphatic ether or hexamethylphosphoramide or in a mixture thereof, or when the alcoholate of the compound of the general formula (II) or (III) is formed with an alkali metal tertiary alcoholate, the corresponding tertiary alcohol or a mixture thereof with the above solvents is used as solvent, or

b) optionally substituted 1-{ω-[bis[phenyl]-methoxy]-alkyl}-piperazines of the general formula

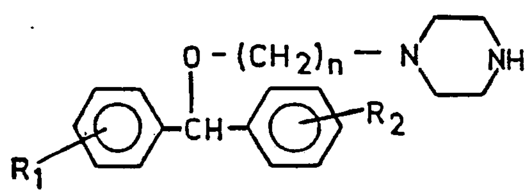

IV,

wherein

$R_1$ and $R_2$ have the meanings given in claims 1 to 4, and

*n* is 2 or 3,

are reacted with optionally substituted [di-(phenyl)]-[ω-(halo)-alkoxy]-methanes, [di-(phenyl)]-[ω-(alkylsulphonyloxy)-alkoxy]-methanes or [di-(phenyl)]-[ω-(arylsulphonyloxy)-alkoxy]-methanes of the general formula

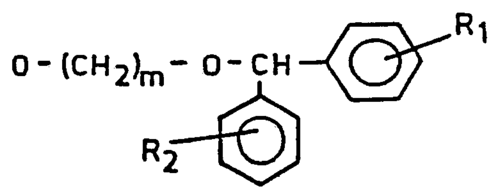

V

wherein

$R_1$ and $R_2$ have the meanings given in claims 1 to 4,

Q is a halogen atom, an alkylsulphonyloxy radical or an arylsulphonyloxy radical, and

m is 2 or 3,

with the proviso that *m* is different from *n*,

the reaction of the monosubstituted piperazine of the general formula (IV) and the compound of the general formula (V) being carried out in an inert organic solvent, namely in an aromatic hydrocarbon, an ether, a ketone, an acid amide, an ester, a lower alkanol or a halogenated hydrocarbon, and the reaction temperature being maintained at between 40°C and the boiling point of the reaction mixture, or

c) optionally substituted benzhydryl halides, benzhydrols or alcoholates thereof of the general formula II are reacted with optionally substituted 1-{ω-[bis-(phenyl)-methoxy]-alkyl}-4-{ω-(halo)-alkyl}-piperazines, -4-{ω-(alkylsulphonyloxy)-alkyl}-piperazines, -4-{ω-(arylsulphonyloxy)-alkyl}-piperazines, -4-{ω-(hydroxy)-alkyl}-piperazines or -4-{ω-(metaloxy)-alkyl}-piperazines of the general formula

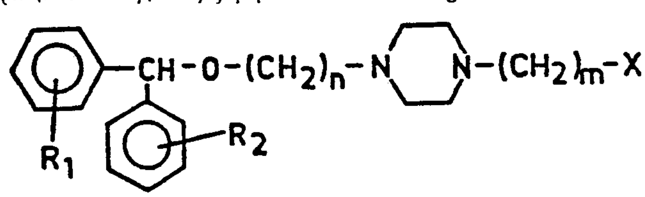

VI,

wherein

$R_1$ and $R_2$ have the meanings given in claims 1 to 4, and

X, *m*, and *n* have the meanings given above, with the proviso that

α) X is a hydroxy group or a radical of the general formula —OM when Y is a halogen atom, or

β) X is a halogen atom, an alkylsulphonyloxy radical or an arylsulphonyloxy radical when Y is a hydroxy group or a radical of the general formula —OM, and also

τ) *m* is different from *n*,

the reaction conditions maintained under a) being maintained, and, in a manner known *per se*, optionally the 1-{2'-[bis-(phenyl)-methoxy]-ethyl}-4-{3'-[bis-(phenyl)-methoxy]-propyl}-piperazines of the general formula I obtained in accordance with one of variants a) to c) are converted into acid addition salts with inorganic or organic acids and/or the resulting acid addition salts of the 1-{2'-[bis-(phenyl)-methoxy]-ethyl}-4-{3'-[bis-(phenyl)-methoxy]-propyl}-piperazines of the general formula I are converted into the corresponding free 1-{2'-[bis-(phenyl)-methoxy]-ethyl}-4-{3'-[bis-(phenyl)-methoxy]-propyl}-piperazine bases of the general formula I or into other acid addition salts and/or optionally the resulting racemic 1-{2'-[bis-(phenyl)-methoxy]-ethyl}-4-{3'-[bis-(phenyl)-methoxy]-propyl}-piperazines of the general formula I or their acid addition salts are cleaved or the corresponding optically active compounds are racemised to form stereoisomers.

7. Medicaments, characterised by a content of one or more compounds according to claims 1 to 5 as active ingredient(s) optionally together with one or more customary pharmaceutical carriers and/or adjuncts.

**Claims for the Contracting State: AT**

1. A process for the preparation of optionally substituted 1-{2'-[bis-(phenyl)-methoxy]-ethyl}-4-{3'-[bis-(phenyl)-methoxy]-propyl}-piperazines of the general formula

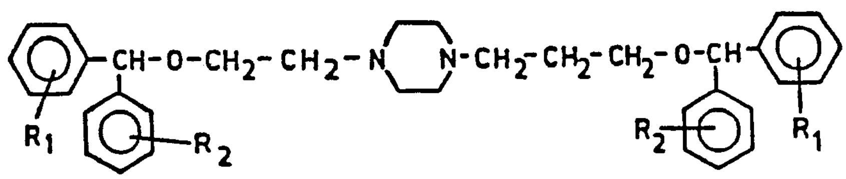

I,

wherein

$R_1$ and $R_2$, which may be identical or different, represent hydrogen atoms, halogen atoms, alkyl radicals having from 1 to 4 carbon atoms, trihalomethyl radicals or alkoxy radicals having from 1 to 4 carbon atoms, and their acid addition salts and optically active isomers and solvates, characterized in that

a) optionally substituted benzhydryl halides, benzhydrols or alcoholates thereof of the general formula

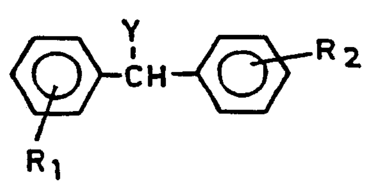

II,

wherein

$R_1$ and $R_2$, have the meanings given in claims 1 to 4, and

Y is a halogen atom, a hydroxy group or a radical of the formula —OM, in which M is an alkali metal atom, are reacted with optionally substituted 1-[2'-(halo)-ethyl]-, 1-[2'-(alkylsulphonyloxy)-ethyl]-, 1-[2'-(arylsulphonyloxy)-ethyl]-, 1-2'-(hydroxy)-ethyl]- or 1-[2'-(metaloxy)-ethyl]-4-(3''-(halo)-propyl]-piperazines, -4-[3''-(alkylsulphonyloxy)-propyl]-piperazines, -4-[3''-(arylsulphonyloxy)-propyl]-piperazine, -4-[3''-(hydroxy)-propyl]-piperazines or -4-[3''-(metaloxy)-propyl]-piperazines of the general formula

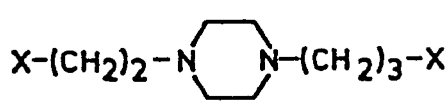

III,

wherein

X is a halogen atom, an alkylsulphonyloxy radical, an arylsulphonyloxy radical, a hydroxy group or a radical of the general formula —OM, wherein

M has the meaning given above, with the proviso that

α) X is a hydroxy group or a radical of the general formula —OM where Y is a halogen atom, or

β) X is a halogen atom, an alkylsulphonyloxy radical or an arylsulphonyloxy radical when Y is a hydroxy group or a radical of the general formula —OM,

and when one of the substituents Y and X is a group of the formula —OM and the other is a halogen atom, the reaction is carried out in an anhydrous medium in a neutral organic solvent, namely an aliphatic or aromatic hydrocarbon, an aliphatic or cycloaliphatic ether or hexamethylphosphoramide or in a mixture

thereof, or when the alcoholate of the compound of the general formula (II) or (III) is formed with an alkali metal tertiary alcoholate, the corresponding tertiary alcohol or a mixture thereof with the above solvents is used as solvent, or

b) optionally substituted 1-{ω-[bis[phenyl]-methoxy]-alkyl}-piperazines of the general formula

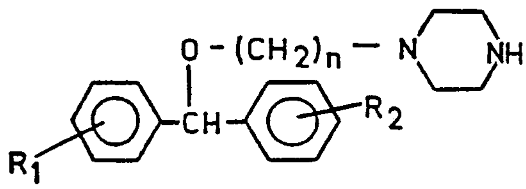

IV,

wherein

$R_1$ and $R_2$ have the meanings given in claims 1 to 4, and

*n* is 2 or 3,

are reacted with optionally substituted [di-(phenyl)]-[ω-(halo)-alkoxy]-methanes, [di-(phenyl)]-[ω-(alkylsulphonyloxy)-alkoxy]-methanes or [di-(phenyl)]-[ω-(arylsulphonyloxy)-alkoxy]-methanes of the general formula

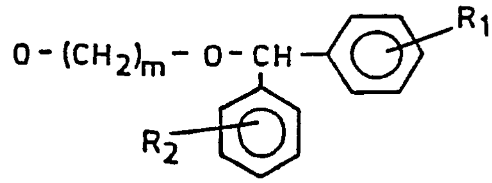

V

wherein

$R_1$ and $R_2$ have the meanings given in claims 1 to 4,

Q is a halogen atom, an alkylsulphonyloxy radical or an arylsulphonyloxy radical, and

*m* is 2 or 3,

with the proviso that *m* is different from *n*,

the reaction of the monosubstituted piperazine of the general formula (IV) and the compound of the general formula (V) being carried out in an inert organic solvent, namely in an aromatic hydrocarbon, an ether, a ketone, an acid amide, an ester, a lower alkanol or a halogenated hydrocarbon, and the reaction temperature being maintained at between 40°C and the boiling point of the reaction mixture, or

c) optionally substituted benzhydryl halides, benzhydrols or alcoholates thereof of the general formula II are reacted with optionally substituted 1-{ω-[bis-(phenyl)-methoxy]-alkyl}-4-{ω-(halo)-alkyl}-piperazines, -4-{ω-(alkylsulphonyloxy)-alkyl}-piperazines, -4-{ω-(arylsulphonyloxy)-alkyl}-piperazines, -4-{ω-(hydroxy)-alkyl}-piperazines or -4-{ω-(metaloxy)-alkyl}-piperazines of the general formula

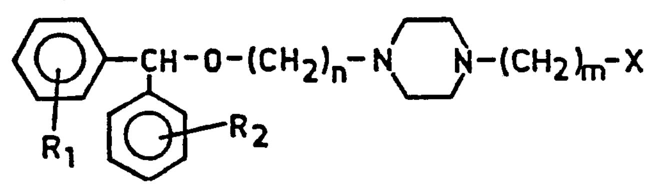

VI,

wherein

$R_1$ and $R_2$ have the meanings given in claims 1 to 4, and

X, *m*, and *n* have the meanings given above, with the proviso that

α) X is a hydroxy group or a radical of the general formula —OM when Y is a halogen atom, or

β) X is a halogen atom, an alkylsulphonyloxy radical or an arylsulphonyloxy radical when Y is a hydroxy group or a radical of the general formula —OM, and also

τ) *m* is different from *n*,

the reaction conditions maintained under a) being maintained, and, in a manner known *per se*, optionally the 1-{2'-[bis-(phenyl)-methoxy]-ethyl}-4-{3'-[bis-(phenyl)-methoxy]-propyl}-piperazines of the general formula I obtained in accordance with one of variants a) to c) are converted into acid addition salts with inorganic or organic acids and/or the resulting acid addition salts of the 1-{2'-[bis-(phenyl)-methoxy]-ethyl}-4-{3'-[bis-(phenyl)-methoxy]-propyl}-piperazines of the general formula I are converted into the corresponding free 1-{2'-[bis-(phenyl)-methoxy]-ethyl}-4-{3'-[bis-(phenyl)-methoxy]-propyl}-piperazine bases of the general formula I or into other acid addition salts and/or optionally the resulting racemic 1-{2'-[bis-(phenyl)-methoxy]-ethyl}-4-{3'-[bis-(phenyl)-methoxy]-propyl}-piperazines of the general formula I or their acid addition salts are cleaved or the corresponding optically active compounds are racemised to form stereoisomers.

2. A process according to claim 1, characterised in that optionally substituted 1-{2'-[bis-(phenyl)-methoxy]-ethyl}-4-{3'-[bis-(phenyl)-methoxy]-propyl}-piperazines are prepared wherein the halogen

atom(s) which $R_1$ and/or $R_2$ may represent and/or the halogen atoms of the trihalomethyl radical(s) which $R_1$ and/or $R_2$ may represent is(are) fluorine and/or chlorine.

3. A process according to claim 1 or 2, characterised in that optionally substituted 1-{2′-[bis-(phenyl)-methoxy]-ethyl}-4-{3′-[bis-(phenyl)-methoxy]-propyl}-piperazines are prepared, wherein the lower alkyl radical(s) which $R_1$ and/or $R_2$ may represent is(are) such (a) radical(s) containing 1 or 2 carbon atoms.

4. A process according to claim 1 or 2, characterised in that optionally substituted 1-{2′-[bis-(phenyl)-methoxy]-ethyl}-4-{3′-[bis-(phenyl)-methoxy]-propyl}-piperazines are prepared wherein the lower alkoxy radical(s) which $R_1$ and/or $R_2$ may represent is(are) such (a) radical(s) containing 1 or 2 carbon atoms.

5. A process according to claim 1, characterised in that 1-[2′-<bis-{4′′-(fluoro)-(phenyl}-methoxy>-ethyl]-4-[3′′′-<bis-{4′′′′-(fluoro)-phenyl}-methoxy>-propyl]-piperazine,

1-[2′-<{4′′-(methoxy)-phenyl}-{3′′′-(trifluoromethyl)-phenyl}-methoxy>-ethyl]-4-[3′′′′-<{4′′′′′-(methoxy)-phenyl}-{3′′′′′′-(trifluoromethyl)-phenyl}-methoxy>-propyl]-piperazine,

1-[2′-<bis-{4′′-(chloro)-phenyl}-methoxy>-ethyl]-4-[3′′′-<bis-{4′′′′-(chloro)-phenyl}-methoxy>-propyl]-piperazine,

1-[2′-<{2′′-(methyl)-phenyl}-{4′′′-(fluoro)-phenyl}-methoxy>-ethyl]-4-[3′′′′-<{2′′′′′-(methyl)-phenyl}-{4′′′′′′-(fluoro)-phenyl}-methoxy>-propyl]-piperazine, and 1-[2′-<di(phenyl)-methoxy>-ethyl]-4-[3′′-<di-(phenyl)-methoxy>-propyl]-piperazine, and their acid addition salts are prepared.